(19) Europäisches Patentamt
European Patent Office
Office européen des brevets

(11) Publication number: **0 415 371 A2**

(12) **EUROPEAN PATENT APPLICATION**

(21) Application number: **90116512.6**

(22) Date of filing: **28.08.90**

(51) Int. Cl.5: **C08K 13/02**, C08K 3/32, C08K 5/3492, C08K 5/49, C08K 5/00, //(C08K13/02,3:32, 5:3492),(C08K5/00,5:49,5:3492)

(30) Priority: **28.08.89 IT 2156289**
**27.03.90 IT 1983990**

(43) Date of publication of application:
**06.03.91 Bulletin 91/10**

(84) Designated Contracting States:
**AT BE CH DE DK ES FR GB LI NL SE**

(71) Applicant: **MINISTERO DELL' UNIVERSITA E DELLE RICERCA SCIENTIFICA E TECNOLOGICA**
**76, Lungotevere Thaon di Revel**
**I-00196 Roma(IT)**

(72) Inventor: **Cipolli, Roberto**
**33, V. le A. Volta**
**28100 Novara(IT)**
Inventor: **Masarati, Enrico**
**321, Via Pianello**
**I-29010 Castelnovo Valtidone, Piacenza(IT)**
Inventor: **Nucida, Gilberto**
**14, Via Mazzini**
**I-20098 S. Giuliano Milanese, Milan(IT)**
Inventor: **Pirozzi, Mario**
**22, Via G. Di Vittorio**
**I-20097 S. Donato Milanese, Milan(IT)**
Inventor: **Oriani, Roberto**
**22, Via M. Ortigara**
**20137 Milan(IT)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

(54) **Self-extinguishing polymeric compositions.**

(57) Described are self-extinguishing polymeric compositions comprising:
(a) from 89 to 45 parts by weight of thermoplastic polymer, or of polymer endowed with elastomeric properties;
b) from 8 to 30 parts by weight of one or more ammonium and/or amine phosphate(s) and/or phosphonate(s);
(c) from 3 to 25 parts by weight of one or more 2,4,6-triamino-1,3,5-triazine derivatives of general formula (I):

EP 0 415 371 A2

(I)

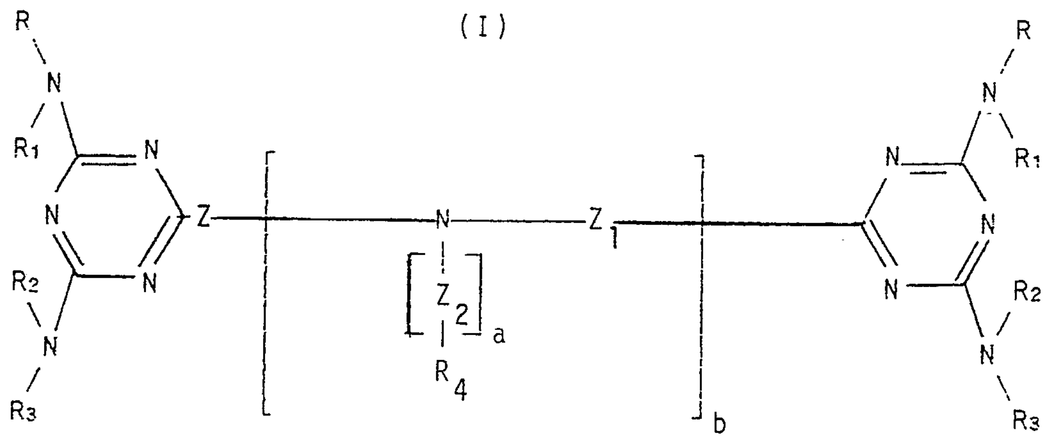

wherein the groups R to $R_4$, Z, $Z_1$ and $Z_2$ and the subscripts a and b are as defined in claim 1.

## SELF-EXTINGUISHING POLYMERIC COMPOSITIONS

The present invention relates to self-extinguishing compositions based on thermoplastic polymers and/or polymers endowed with elastomeric properties, in particular olefinic polymers or copolymers, which compositions contain specific triazine compounds in combination with ammonium and/or amine phosphates and/or phosphonates.

Several methods of reducing or eliminating the combustibility of polymers are known in the art. Some of said methods are based on the use of metal compounds, in particular on antimony, bismuth or arsenic, in combination with partially halogenated, thermally unstable organic compounds, such as chlorinated paraffin waxes.

Other methods are based on the use of substances capable of yielding intumescence. The formulations of the intumescent type generally are composed of the polymer and at least three main additives, i.e., an essentially phosphorus-containing additive, whose purpose is to form, during the combustion, an impermeable, semi-solid and vitreous layer essentially composed of polyphosphoric acid and to activate the process of formation of intumescence; a second additive, containing nitrogen, which is to serve as foaming agent; and a third, carbon-containing additive, Which acts as a carbon donor in order to allow an insulating cellular carbonaceous layer ("char") to be formed between the polymer and the flame.

Examples of intumescent formulations of said type may be found in US-A-3,810,862 (Phillips Petroleum Co.; based on melamine, pentaerythritol and ammonium polyphosphate); US-A-4,727,102 (Vamp S.r.l.; based on melamine cyanurate, a hydroxyalkyl derivate of isocyanuric acid and ammonium polyphosphate); and WO-85/05626 (Plascoat U.K. Limited; on the basis of various phosphorus and nitrogen compounds among which, in particular, a combination of melamine phosphate, pentaerythritol and ammonium polyphosphate may be mentioned).

In more recent formulations, together with an organic or inorganic phosphorus compound, a nitrogen-containing organic compound was used, generally consisting of an aminoplastic resin obtained by means of condensation of urea, melamine or dicyandiamide with formaldehyde.

Examples of formulations containing two additives are those described in US-A-4,504,610 (Montedison S.p.A.; based on oligomeric derivates of 1,3,5-triazine and ammonium polyphosphate) and EP-A-14,463 (Montedison S.p.A.; based on organic compounds selected from benzylguanamine and reaction products of aldehydes and several cyclic nitrogen compounds, in particular benzylguanamine-formaldehyde copolymers, and ammonium polyphosphate).

Self-extinguishing compositions can also be obtained by using single-component additives, which contain in their organic molecule both nitrogen and phosphorus atoms, as disclosed in US-A-4,201,705 (Borg-Wagner Corp.).

These intumescent flame retardant systems endow the polymers to which they are added with the property of forming a carbonaceous residue when they burn or are exposed to a flame. This kind of flame-retardant system offers numerous advantages, i.e., absence of corrosion phenomena in the machinery in which the polymers are processed, a lower emission of smokes as compared to systems containing metal compounds and halogenated hydrocarbons, and, above all, the possibility of endowing the polymers with satisfactory flame-proof properties with a smaller amount of total additive and therefore without excessively impairing the mechanical properties thereof.

It has now surprisingly been found that polymeric compositions endowed with extremely good flame-proof properties are obtained when certain derivatives of 2,4,6-triamino-1,3,5-triazine are added thereto, the effectiveness of said derivatives being even higher than that of products known from the prior art.

More specifically, the compositions according to the present invention comprise:

(a) from 89 to 45 parts by weight of thermoplastic polymer and/or of polymer endowed with elastomeric properties;

(b) from 8 to 30 parts, and preferably from 12 to 30 parts by weight of one or more ammonium and/or amine phosphates and/or phosphonates;

(c) from 3 to 25 parts, and preferably from 6 to 20 parts by weight, of at least one 2,4,6-triamino-1,3,5-triazine derivative of general formula (I):

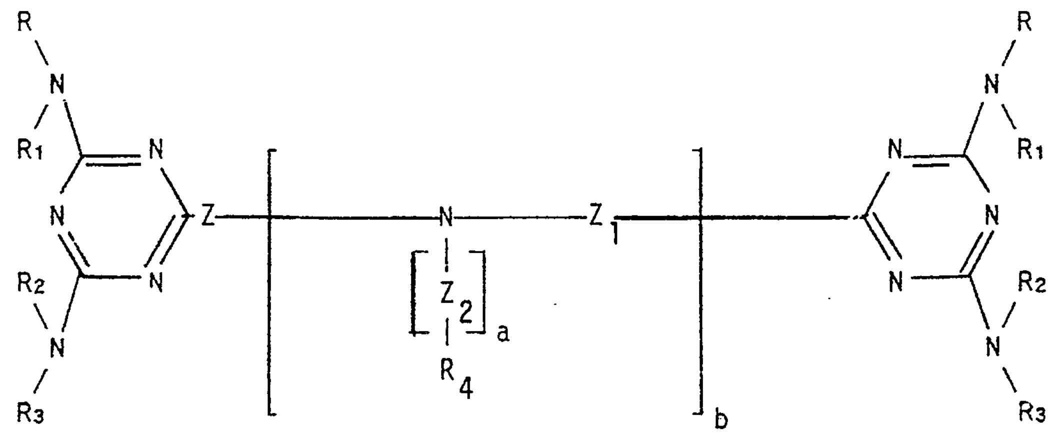

(I)

wherein the radicals R to $R_3$, equal to or different from 80 one another and having the same or different meanings in each triazine ring, are selected from H; $(C_1-C_{18})$-alkyl; $(C_2-C_8)$-alkenyl; $(C_6-C\ 6)$-cycloalkyl and $(C_6-C_{16})$-alkylcycloalkyl, optionally substituted with one or more hydroxy and/or $(C_1-C_4)$-hydroxyalkyl groups;

$-[-C_nH_{2n}-]-O-R_5$ ; and

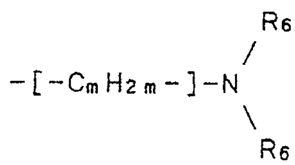

;

wherein:

n = an integer of from 2 to 8, preferably from 2 to 4;

m = an integer of from 2 to 6;

$R_5$ = H; $(C_1-C_8)$-alkyl; $(C_2-C_6)$-alkenyl; $[-C_pH_{2p}-]-O-R_7$,

wherein P is an integer of from 1 to 4 and $R_7$ is H or a $(C_1-C_4)$-alkyl group; $(C_6-C_{12})$-cycloalkyl or $(C_6-C_{12})$-alkylcycloalkyl;

preferably H or $(C_1-C_4)$-alkyl;

the radicals $R_6$, equal to or different from one another, are selected from H; $(C_1-C_8)$-alkyl; $(C_2-C_6)$-alkenyl; $(C_6-C_{12})$-cycloalkyl or $(C_6-C_{12})$-alkylcycloalkyl; and $(C_1-C_4)$-hydroxyalkyl;

or the moiety $N(R_6)_2$ is replaced by an N-heterocyclic radical linked to the alkyl chain through the nitrogen atom and optionally containing another heteroatom (preferably selected from O, N and S);

or in general formula (I) at least one of the moieties

$NRR_1$ and $NR_2R_3$

is replaced by an N-heterocyclic radical linked to the triazine ring through the nitrogen atom and optionally containing another heteroatom (preferably selected from O, N and S);

a is 0 or 1;

b is 0 or an integer of from 1 to 5;

$R_4$ is hydrogen or a group of general formula

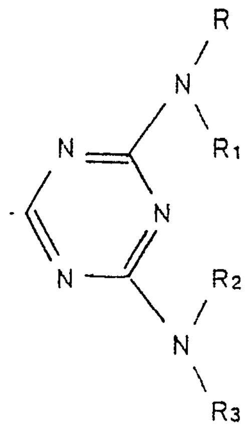

and its meaning can be different in each repeating unit;
when b is zero:
Z is a divalent radical selected from radicals of the following formulae:

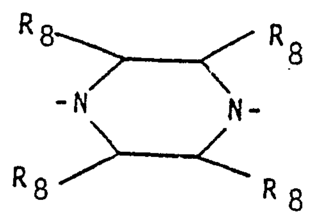

(II)

where the groups $R_8$, the same or different from one another, represent hydrogen or $(C_1$-$C_4)$-alkyl;

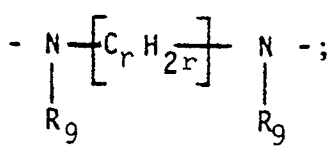

(III)

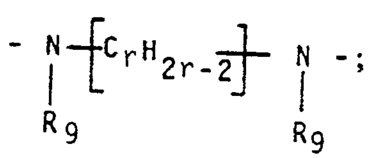

(IV)

where r is an integer of from 2 to 14 and $R_9$ is hydrogen; $(C_1$-$C_4)$-alkyl; $(C_2$-$C_6)$-alkenyl; or $(C_1$-$C_4)$-hydroxyalkyl;

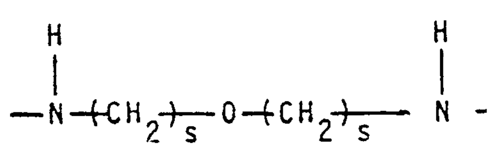

(V)

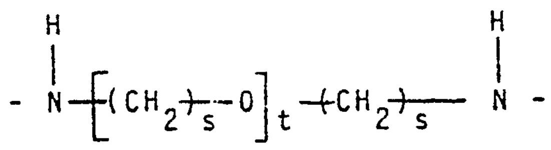

(VI)

where s is an integer of from 2 to 5 and t is an integer of from 1 to 3;

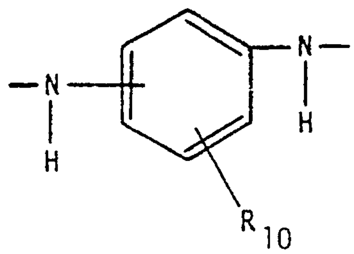

(VII)

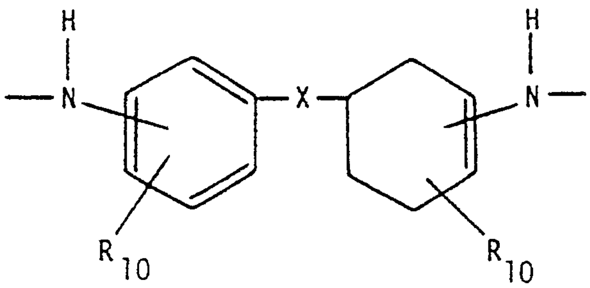

(VIII)

where:
X represents a direct bond (C-C); O; S; S-S; SO; $SO_2$; NH; $NHSO_2$; NHCO; N=N; $CH_2$;
$R_{10}$ is hydrogen; hydroxy; ($C_1$-$C_4$)-alkyl; or ($C_1$-$C_4$)-alkoxy;

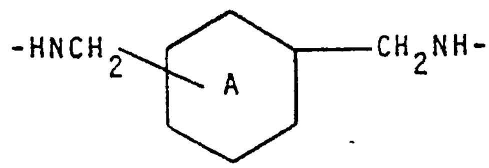

(IX)

where A is a saturated or unsaturated ring;

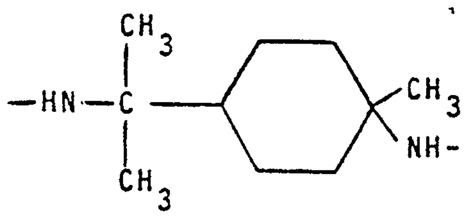

(X)

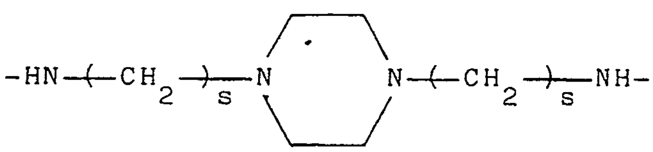

(XI)

where s has the meaning defined hereinabove;
when b is an integer of from 1 to 5:
the group:

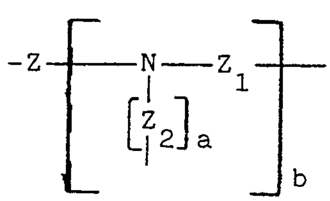

is a polyvalent radical represented by one of the following formulae:

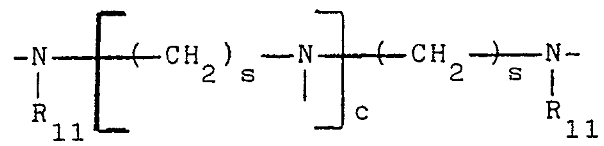 (XII)

where:
$R_{11}$ is hydrogen or ($C_1$-$C_4$)-alkyl;
c is an integer of from 1 to 5;
the subscripts s, equal to or different from each other, have the meaning defined hereinabove; and

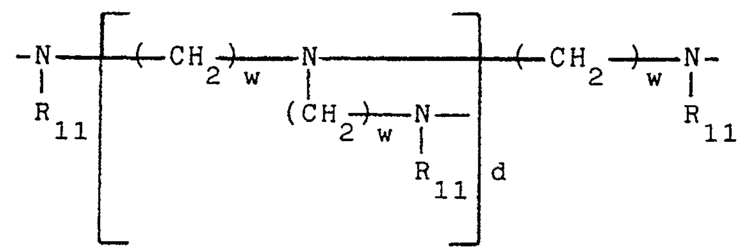 (XIII)

where:
$R_{11}$ has the meaning defined hereinabove;
w is an integer of from 2 to 4; and
d is 1 or 2.

Derivatives having an asymmetrical structure in that the radicals R to $R_3$ have different meanings in each triazine ring are also meant to be included in the above definition.

The above compounds of general formula (I), besides having a simple structure (polycondensates of 2,4,6-triamine-1,3,5-triazine), are particularly stable when heated and therefore retain a high activity as flame-retardants also after the hot-fabrication processes of the polymeric compositions which contain them.

The compositions according to the present invention furthermore show the advantage that in the case of a fire they give rise to a very moderate and not obscuring smoke emission.

Specific examples of radicals R, $R_1$, $R_2$ and $R_3$ in the above general formula (I) are: methyl; ethyl; propyl; isopropyl; n-butyl; isobutyl; tert -butyl; n-pentyl; isopentyl; n-hexyl; tert-hexyl; octyl; tertoctyl; decyl; dodecyl; octadecyl; ethenyl; propenyl; butenyl; isobutenyl; hexenyl; octenyl; cyclohexyl; propylcyclohexyl: butylcyclohexyl; decyloyclohexyl; hydroxycyclohexyl; hydroxyethylcyclohexyl; 2-hydroxyethyl; 2-hydroxypropyl; 3-hydroxypropyl; 3-hydroxybutyl; 4-hydroxybutyl; 3-hydroxypentyl; 5-hydroxypentyl; 6-hydroxyhexyl; 3-hydroxy-2,5-dimethylhexyl; 7-hydroxyheptyl; 7-hydroxyootyl; 2-methoxyethyl; 2-methoxypropyl; 3-methoxypropyl; 4-methoxybutyl; 6-methoxyhexyl; 7-methoxyheptyl; 7-methoxyoctyl; 2-ethoxyethyl; 3-ethoxypropyl; 4 ethoxy butyl; 3-propoxypropyl; 3-butoxypropyl; 4 butoxybutyl; 4-isobutoxybutyl; 5-propoxypentyl; 2-cyclohexyloxyethyl

2-ethenyloxyethyl; 2-(N N-dimethylamino)ethyl; 3-(N,N-dimethylamino)propyl; 4-(N,N-dimetnylamino)-butyl; 5-(N,N-dimethylamino)pentyl; 4-(N,N-diethylamino)butyl; 5-(N,N-diethylamino)pentyl; 5-(N,N diisopropylamino)pentyl; 3-(N-ethylamino)propyl; 4-(N-methylamino)butyl; 4-(N,N-dipropylamino)butyl; 2-(N,N-diisopropylamino)ethyl; 6-(N-hexenylamino)hexyl; 2-(N-ethenylamino)ethyl; 2-(N-cyclohexylamino)ethyl; 2-(N-2-hydroxyethylamino)ethyl; 2-(2-hydroxyethoxy)ethyl; 2-(2-methoxyethoxy)ethyl; 6-(N-propylamino)-hexyl; etc.

Preferred examples of heterocyclic radicals which can replace the moieties $NRR_1$ and $NR_2R_3$ are: aziridinyl; pyrrolidinyl; piperidinyl; morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2-ethylpiperazinyl; 2,5-diethylpiperazinyl; etc.

Preferred examples of heterocyclic radicals which can replace the moiety $N(R_6)_2$ are: aziridinyl; pyrrolidinyl; piperidinyl; morpholinyl; thiomorpholinyl; piperazinyl; 4-methylpiperazinyl; 4-ethylpiperazinyl; etc.

Specific examples of divalent -Z- radicals are those deriving, by elimination of a hydrogen from each amine group, from the following diamino compounds:

piperazine; 2-methylpiperazine; 2,5-dimethylpiperazine; 2,3,5,6-tetramethylpiperazine; 2-ethylpiperazine; 2,5-diethylpiperazine; 1,2-diaminoethane; 1,3-diaminopropane; 1,4-diaminobutane; 1,5-diaminopentane; 1,6-diaminohexane; 1,8-diaminooctane; 1,10-diaminodecane; 1,12-diaminododecane; N,N'-dimethyl-1,2-diaminoethane; N-methyl-1,3-diaminopropane; N-ethyl-1,2-diaminoethane; N-isopropyl-1,2-diaminoethane; N-(2-hydroxyethyl)-1,2-diaminoethane; N,N'-bis(2-hydroxyethyl)-1,2-diaminoethane; N-(2-hydroxyethyl)-1,3-diaminopropane; N-hexenyl-1,6-diaminohexane; N,N'-diethyl-1,4-diamino-2-butene; 2,5-diamino-3-hexene; 2-aminoethylether; (2-aminoethoxy)methylether; 1,2-bis (2-aminoethoxy)ethane: 1,3-diaminobenzene; 1,4-diaminobenzene; 2,4-diaminotoluene; 2,4-diaminoanisole; 2,4-diaminophenol; 4-aminophenylether; 4,4'-methylenedianiline; 4,4'-diaminobenzanilide: 3-aminophenylsulfone; 4-aminophenylsulfone; 4-aminophenyldisulfide: 4-aminophenylsulfoxide; 1,3-bis-(aminomethyl)benzene: 1,4-bis(aminomethyl)benzene; 1,3-bis-(aminomethyl)cyclohexane; 1,8-diamino-p-menthane; 1,4-bis(2-aminoethyl)piperazine; 1,4-bis(3-aminopropyl)piperazine; 1,4-bis(4-aminobutyl)piperazine; 1,4-bis(5-aminopentyl)piperazine; etc.
Examples of polyvalent radicals of formula

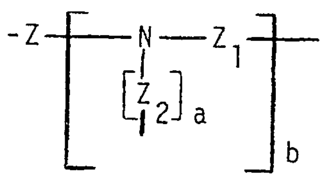

are those which derive, by elimination of a hydrogen atom from each reacted amino group' from the following polyamino compounds:
bis(2-aminoethyl)amine; bis(3-amlnopropyl)amine; bis(4-aminobutyl) amine; bis(5-aminopentyl)amine; bis [2-(N-methylamino)ethyl] amine; 2-N-butyl-bis(2-aminoethyl)amine; bis [3-(N-methylamino)propyl]amine; N-(3-aminopropyl)-1,4-diaminobutane; N-(3-aminopropyl)-1,5-diaminopentane; N-(4-aminobutyl)-1,5-diaminopentane; tris(2-aminoethyl)amine; tris(3-aminopropyl)amine; tris(4-aminobutyl)amine; tris[2-(N-ethylamino)ethyl]-amine; N,N'-bis(2-aminoethyl)-1,2-diaminoethane; N,N'-bis(3-aminopropyl)-1,3-diaminopropane; N,N'-bis(2-aminoethyl) 1,3-diaminopropane; N,N'-bis(3-aminopropyl)-1,2-diami-noethane; N,N'- bis(3-aminopropyl-1,4-diaminobutane; bis [2-(2-aminoethyl)aminoethyl]-amine; N,N'-bis [2-(2-aminoethyl)aminoethyl]-1,2-diaminoethane; N,N'-bis [3-(2-aminoethyl)aminopropyl]-1,2 diaminoethane; N,N,N',N',-tetrakis(2-aminoethyl)-1,2-diaminoethane; etc.

The compounds of formula (I) in which at least one of the moieties $NRR_1$ and $NR_2R_3$ is a $-NH_2$ radical are preferred.

Specific compounds comprised within the scope of general formula (I) which can be advantageously used in the compositions according to the present invention are given in the examples below.

The compounds of general formula (I) may be prepared by reacting, preferably at temperatures of from about 0 to about 10 °C and at a pH value of from 5 to 7, a halide of cyanuric acid, such as, e.g., cyanuric chloride, in a suitable solvent (such as, e.g., acetone, water and methylene chloride) with an amine having the general formula (XIV):

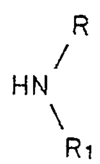

(XIV)

wherein R and $R_1$ have the meanings given above, in the presence or absence (depending on the molar ratio used in the reaction) of an acid acceptor (such as, e.g., NaOH, $NaHCO_3$, $Na_2CO_3$ and triethylamine), resulting in the formation of the intermediate of general formula (XV):

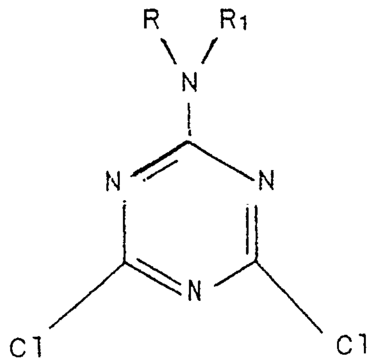

(XV)

Said intermediate, isolated or not isolated from the reaction mixture, may subsequently be reacted, under conditions similar or identical to those specified hereinabove, but preferably at a temperature of from about 10 to about 50 °C, with an amine of general formula (XVI):

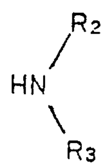

(XVI)

wherein $R_2$ and $R_3$ have the meanings given above, resulting in the formation of the intermediate of general formula (XVII):

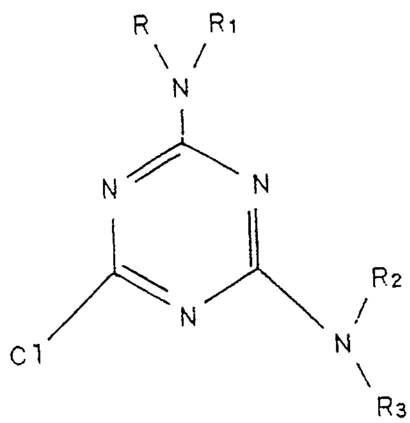

(XVII)

The intermediate (XVII), isolated or not isolated, and in a number of mols smaller than or equal to (2+b), may in turn be reacted, under the same conditions as in the case of the two first reaction steps, but preferably operating at a temperature of from about 70 to about 150 °C (and hence using solvents which are compatible with such temperatures, such as, e.g., water, toluene, xylene and dimethylformamide), with one mol of a polyamine having the general formula (XVIII):

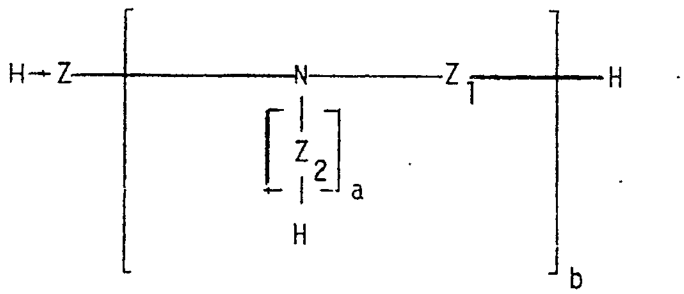

(XVIII)

which corresponds to the structures comprised within the range of general formulae (II) to (XIII); affording the compounds of general formula (I) as the end products.

In case compounds of general formula (I) wherein the groups $NRR_1$ and $NR_2R_3$ are the same are desired, the above process may be carried out by reacting cyanuric chloride with two mols of an amine of general formula (XIV) under the same conditions as disclosed hereinabove in order to obtain the intermediate of general formula (XVII).

An alternative method comprises the reaction of a number of mols lower than or equal to (2+b) of a halide (such as, e.g., the chloride) of cyanuric acid with one mol of a polyamine of general formula (XVIII) as defined above, under the same conditions as disclosed hereinabove, and preferably at a temperature of from about 0 to about 10° C so as to yield the intermediate of general formula (XIX):

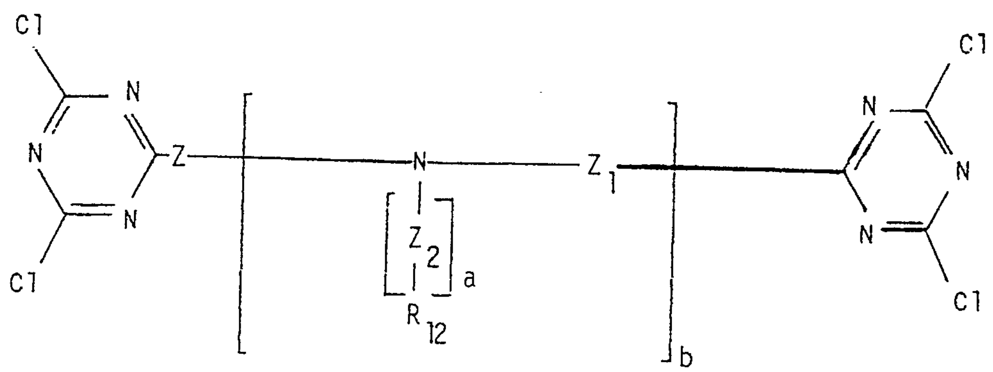

(XIX)

wherein $R_{12}$ is hydrogen or a group of formula

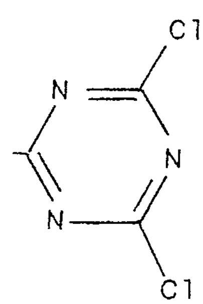

and its meaning can be different in each repeating unit.

Said intermediate, isolated or not isolated, may be reacted:

(a) with a number of mols lower than or equal to (2+b) of an amine of general formula (XIV), preferably at a temperature of from about 40 to about 80° C, in order to yield intermediate of general formula (XX):

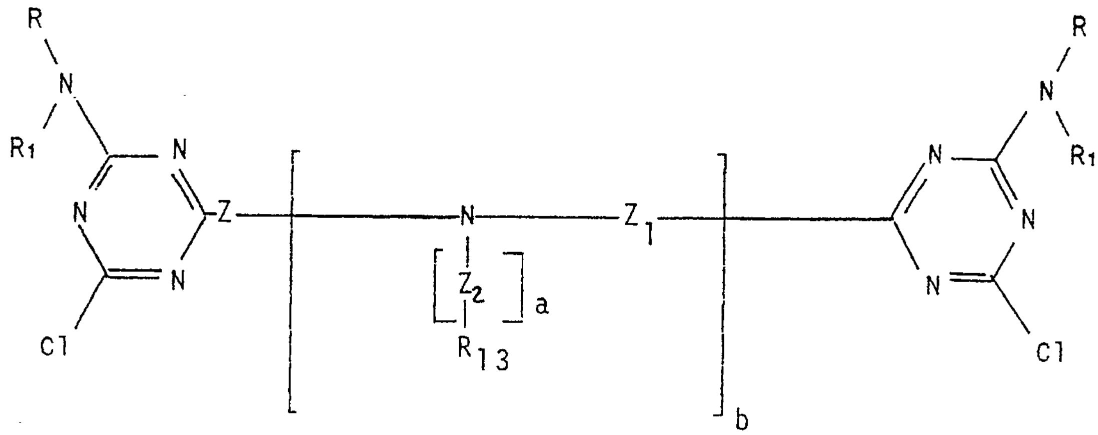

(XX)

wherein $R_{13}$ is hydrogen or a group of the formula

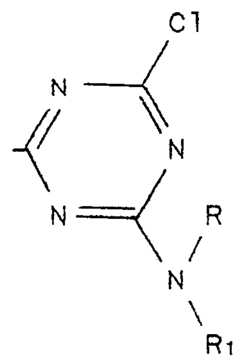

and its meaning can be different in each repeating unit; which intermediate, isolated or not isolated,may be reacted with a number of mols lower than or equal to (2+b) of an amine of general formula (XVI), preferably at a temperature of from about 80 to about 150°C and under conditions similar to those disclosed hereinabove; or

(b) with a number of mols lower than or equal to 2(2+b) of an amine of general formula (XIV), preferably at a temperature of from about 80 to about 150°C, under conditions similar to those disclosed hereinabove.

Among the phosphates, ammonium polyphosphates of the general formula

$(NH_4)_{n+2}P_nO_{3n+1}$

wherein n is an integer equal to, or greater than, 2, are preferred. The molecular weight of the polyphosphates should be high enough to secure a low solubility in water.

For exemplary purposes, n preferably is comprised within the range of from 2 to 500.

The composition of the polyphosphates having the formula indicated hereinabove, in which n is a high enough number, and preferably ranges from 5 to 500, practically corresponds to a metaphosphate of formula

$(NH_4PO_3)_n$.

An example of said polyphosphates is the product known under the trade name "Exolit® 422" (Hoechst), having the composition $(NH_4PO_3)n$ in which n is greater than 50. Another example is the product known under the name "Phos-Chek® P/30" (Monsanto Chemical) which has a similar composition.

Another polyphosphate which can be used advantageously, above all thanks to its low solubility in water, is the product known under the trade name "Exolit® 462" (Hoechst) which is "Exolit® 422" microencapsulated in melamine-formaldehyde resin.

Other phosphates which can be used are those which are derived from amines, such as, e.g., dimethyl ammonium phosphate or diethyl ammonium phosphate, ethylene diamine phosphate, melamine orthophosphate or melamine pyrophosphate.

Good results may also be obtained by using mono- or polyammonium phosphonates selected from the

salts derived from mono- or polyphosphonic acids.

Examples of said acids are:

ethane-1,1 2-triphosphonic acid ethane-2-hydroxy-1,1,2-triphosphonic acid, propane-1,2,3-triphosphonic acid, methylphosphonic acid, ethylphosphonic acid, n-propylphosphonic acid, n-butylphosphonic acid, phenylphosphonic acid, ethane-1-amino-1,1-diphosphonic acid ethane-1-hydroxy-1,1-diphosphonic acid, dodecane-1-hydroxy-1,1-diphosphonic acid, phosphonoacetic acid, 2-phosphonopropionic acid, 3-phosphonopropionic acid, 2-phosphonobutyric acid, 4-phosphonobutyric acid, amine tri-(methylenephosphonic) acid, ethylenediamine tetra(methylenephosphonic) acid, hexamethylene diamine tetra(methylenephosphonic) acid, diethylene triamine penta(methylenephosphonic acid, etc.

Among the polymers which can be used in the compositions according to the present invention, preferred are polymers and copolymers of olefins of general formula

$R' - CH = CH_2$

wherein $R'$ is hydrogen or a ($C_{1-8}$)-alkyl or ($C_{6-10}$)-aryl radical, in particular:

(1) isotactic or prevailingly isotactic polypropylene;

(2) HDPE, LLDPE and LDPE polyethylene;

(3) crystalline copolymers of propylene and minor amounts of ethylene and/or of other alpha-olefins, such as, e.g., 1-butene, 1-hexene, 1-octene, 4-methyl-1-pentene;

(4) heterophasic compositions comprising (A) a fraction constituted by a propylene homopolymer, or by one of the copolymers defined under (3); and (B) a copolymeric fraction constituted by elastomeric copolymers of ethylene and an alpha-olefin, optionally containing minor proportions of one or more dienes, wherein the alpha-olefin is preferably selected from propylene and 1-butene; and

(5) elastomeric copolymers of ethylene and alpha-olefin(s), optionally containing minor proportions of one or more dienes.

Examples of dienes more commonly contained in said elastomeric copolymers are butadiene, ethylidene-norbornene and hexadiene-1,4.

Among the polymers of olefins of general formula $R'-CH=CH_2$ wherein $R'$ is an aryl radical, "crystal" and impact-resistant polystyrene are preferred.

Other examples of polymers which can be used are acrylonitrile/butadiene/styrene terpolymers (ABS); styrene/acrylonitrile copolymers (SAN); polyurethanes (of polyester or polyether grade); poly(ethylene terephthalate); poly(butylene terephthalate); polyamides; etc.

The self-extinguishing compositions according to the present invention can be prepared according to methods known in the art. For example, ammonium and/or amine phosphate and/or phosphonate is first intimately mixed with one or more finely ground nitrogen-containing compounds of general formula (I) (the particles of which generally are smaller than 70 μm) and the thus obtained mixture is added to the polymer in a turbo-mixer, in order to generate a homogeneous mixture which is either extruded or granulated. The thus obtained granular product can be transformed into various finished articles according to any of the well-known moulding techniques.

The fire-retardant additives according to the present invention are also suitable for use in the field of flame-retardant paints. The following examples are illustrative but do not limit the present invention.

Example 1

73.8 g of cyanuric acid chloride, 240 g of acetone and 300 g of ice were charged into a 1 liter reactor equipped with stirrer, thermometer, dropping funnel, reflux cooler and cooling bath.

Under stirring and maintaining a temperature ranging from 0 to 5° C, 39.6 g of cyclohexylamine and 16.0 g of sodium hydroxide dissolved in 40 g of water, were simultaneously fed, in order to maintain a pH of from 5 to 7.

The whole mixture was kept at a temperature of 0°-5° C for a further 2 hours, then the resulting product was filtered and washed with water.

The cake was dried, which afforded 87g of intermediate (XXI)

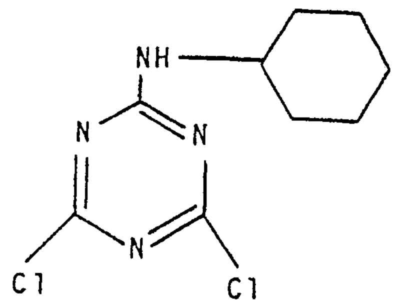

(XXI)

as white crystalline powder having a melting point (m.p.) of 70-71°C and a chlorine content of 28.61% (theoretical value: 28.74%).

A 0.5 liter reactor equipped with stirrer, thermometer, feeding funnel, reflux cooler and heating bath was charged with 74.1 g of intermediate (XXI) and 160 g of acetone.

The mixture was heated to 40°C under stirring until a solution was obtained, then 45 g of aqueous ammonia at 30% by weight were added over 30 minutes, maintaining the temperature at 40°C.

The reaction mass was then heated to 45°C and kept at this temperature for 4 hours.

After cooling, the obtained product was filtered and then it was washed with water on the filter.

After drying in an oven at 80°C, 67 g of intermediate (XXII):

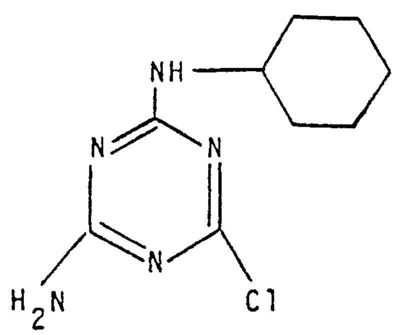

(XXII)

were obtained in the form of a white powder having a m.p. of 180-183°C and a chlorine content of 15.51% (theoretical value: 15.6%).

The structure of compounds (XXI) and (XXII) was confirmed by NMR analyses.

A 1 liter reactor equipped like the preceding reactor was charged with 63.7 g of intermediate (XXII), 400 ml of xylene and 12.1 g of piperazine.

Under stirring, said mixture was heated to 100°C and then, over 4 hours and with the temperature being maintained at 100°C, 11.2 g of sodium hydroxide, dissolved in 20 g of water, were added. The temperature was gradually raised while azeotropically eliminating water, until reaching the solvent boiling temperature.

The whole mixture was kept under reflux for 12 hours, then the mass was cooled to room temperature and the resulting product was filtered. The cake was thoroughly pressed and then repeatedly washed with water.

After drying, 62.5 g of product of formula

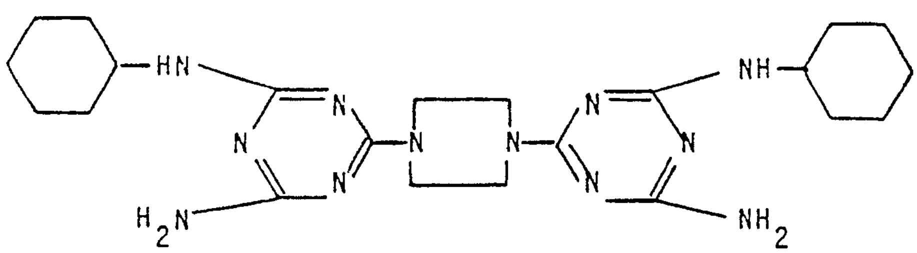

in the form of a white crystalline powder having m.p. of 265 -268°C were obtained.

Example 2

A 3 liter reactor equipped with stirrer, thermometer, feeding funnel, reflux cooler and heating bath was charged with 184.5 g of cyanuric acid chloride and 650 g of acetone.

Under stirring, the mixture was heated to 40 ° C until a solution was obtained. Then, maintaining the temperature at 40 ° C, 284 g of aqueous ammonia at 30% by weight were added over 1.5 hours.

Thereafter the resulting mixture was heated to 45 ° C and this temperature was maintained for 4 hours.

After cooling, the resulting product was filtered and washed with water on the filter.

After drying in an oven at 50 ° -60 ° C and under vacuum, there were obtained 113 g of intermediate-(XXIII):

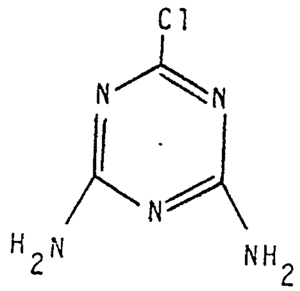

(XXIII)

in the form of a white crystalline, non-meltable powder having a chlorine content of 24.2% (theoretical: 24.4%).

The structure of this compound was also confirmed by infrared spectroscopic analysis.

A 1 liter reactor equipped like the preceding one was charged with 400 ml of xylene, 58.2 g of intermediate (XXIII) and 17.2 g of piperazine.

The mass was heated to 100 ° C and this temperature was maintained for 2 hours.

16 g of solid sodium hydroxide were then added and the whole mixture was brought to reflux, said condition being maintained for about 20 hours. Then the mass was cooled to room temperature and filtered.

The filter cake was repeatedly washed with water and then dried.

54.2 g of product of formula

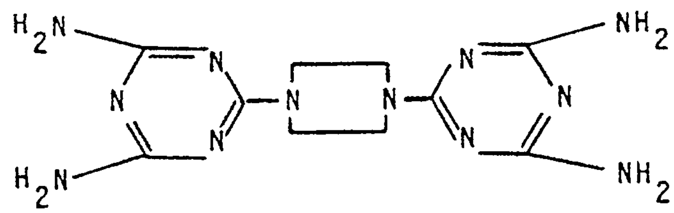

in the form of a white crystalline powder having a m. p. higher than 300 ° C were obtained.

Example 3

The same 3 liter apparatus described in example 2, but initially equipped with a cooling bath, was charged with 184.5 g of cyanuric acid chloride and 1,300 ml of methylene chloride.

Wile cooling from the outside, 87.2 g of morpholine and 40 g of sodium hydroxide, dissolved in 150 g of water, were simultaneously fed over 3 hours, thus maintaining a pH of from 5 to 7 and a temperature of 0 to 3 ° C.

The temperature was maintained at 0 to 3 ° C for a further 3 hours, whereafter the aqueous phase was separated.

By distillation of methylene chloride, 230 g of intermediate (XXIV):

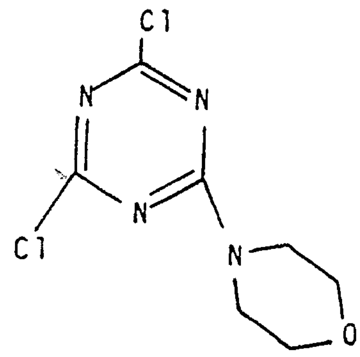

(XXIV)

in the form of a white crystalline powder having a m.p. of155-157°C and a chlorine content of 29.87% (theoretical value: 30.21%) were obtained.

A 0.5 liter reactor, equipped as described in example 2, was charged with 200 g of a solution at 30% by weight of ammonia and 70.5 g of intermediate (XXIV).

The mixture was heated to 50°C and this temperature was o maintained for 7 hours. The whole mass was then allowed to cool to room temperature and the resulting product was filtered and washed with water.

The cake was dried, thereby obtaining 58 g of intermediate (XXV):

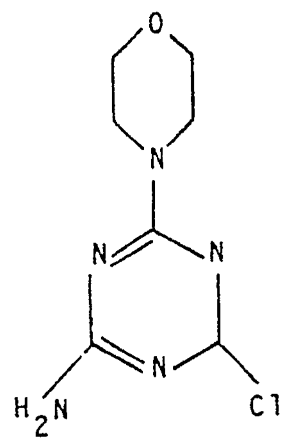

(XX V)

in the form of a white crystalline powder having a m.p. of 189-191°C and a chlorine content of 16.28% (theoretical value: 16.47%).

The structure of compounds(XXIV) and (XX V) was confirmed by infrared spectroscopic analysis.

A 1 liter reactor equipped like that described above was charged with 400 ml of ortho-dichlorobenzene, 53.9 g of intermediate (XXV) and 14.5 g of hexamethylenediamine.

The mixture was heated to 100°C and this temperature was maintained for 2 hours. 10 g of sodium hydroxide were then added and the temperature was raised to 140°C. The mass was kept at 140°C for 16 hours, whereafter it was cooled to room temperature and the resulting product was filtered and repeatedly washed with water.

After drying, 62.3 g of product of formula

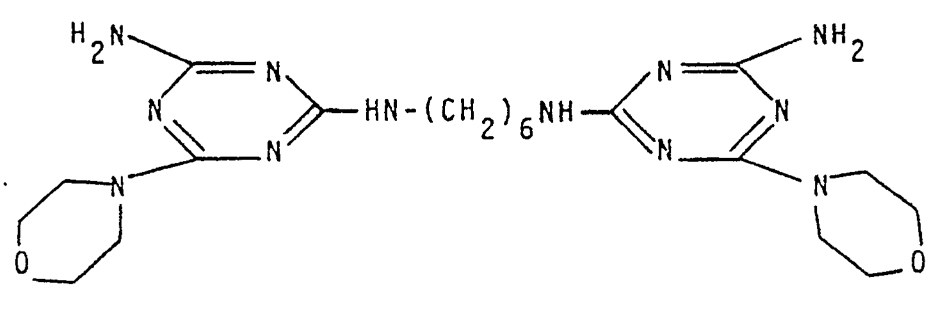

were obtained in the form of a white crystalline powder having a m. p. of 267-269°C.

Example 4

Into the same 1 liter reactor as used in example 3 there were placed 400 ml of xylene, 53.9 g of intermediate (XXV) and 10.8 g of para-phenylenediamine.

Operating according to the same modalities as described in example 3, there were obtained, after drying, 55.7 g of product of formula

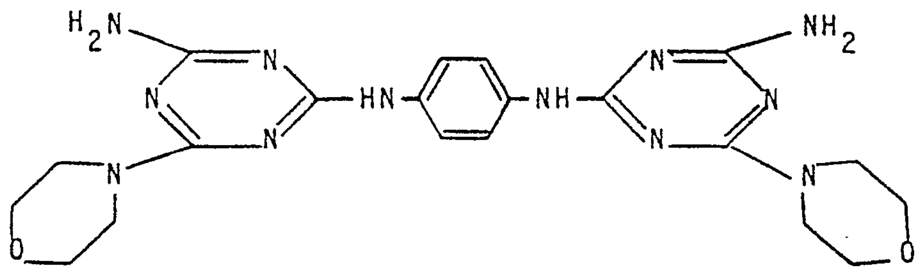

in the form of a white crystalline powder having a m. p. higher than 300° C.

Example 5

A 2 liter reactor, equipped like that used in the preceding examples, was charged with 129 g of cyanuric acid chloride and 1,000 ml of methylene chloride.

While cooling from the outside, 40 g of 3-amino-1-propene, dissolved in 150 g of water, were added to the solution maintained at 0-2° C over 90 minutes.

Maintaining the temperature at 0-2° C, 28 g of sodium hydroxide in 100 g of water were added over 2 hours. The whole mixture was stirred for 2 hours at a temperature of 3-5° C, whereafter the aqueous phase was separated.

By distillation of methylene chloride, 137 g of intermediate (XXVI):

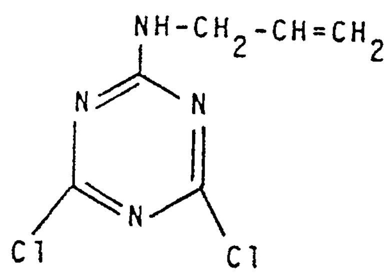

(XXVI)

in the form of a white crystalline powder having a m.p.of 70-72° C and a chlorine content of 34.52% (theoretical value: 34.63%) were obtained.

Into the same apparatus described hereinbefore, there were placed 200 g of aqueous ammonia at 30% by weight and 500 g of water.

The mixture was heated to 40° C and then, maintaining the temperature at 40° C, 123 g of intermediate (XXVI) were added over 30 minutes.

The temperature was brought to 45° C and was maintained at said value for about 6 hours.

Upon completion of the reaction, the whole mixture was cooled to room temperature and the obtained product was filtered. The product was then washed with water and dried.

Obtained were 104 g of intermediate (XXVII)

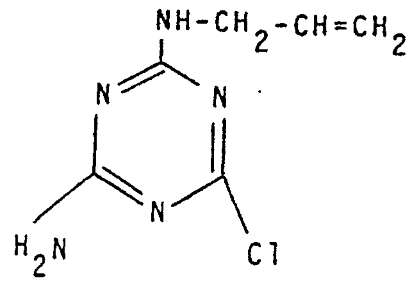

(XXVII)

as a white crystalline powder having a m.p. of 168 -170 °C and a chlorine content of 19.03% (theoretical value: 19.14%).

The structure of intermediates (XXVI) and (XXVII) was confirmed by NMR analysis.

Into a 1.0 liter reactor equipped as described above, there were charged 450 ml of xylene, 55.7 g of intermediate (XXVII) and 17.1 g of 2,5-dimethylpiperazine.

The resulting mass was heated to 100 °C for 2 hours, then 12 g of solid sodium hydroxide were added and the whole mixture was brought to reflux.

Reflux was maintained for 18 hours, then the procedure of the preceding examples was followed.

There were obtained 56.3 g of product of formula

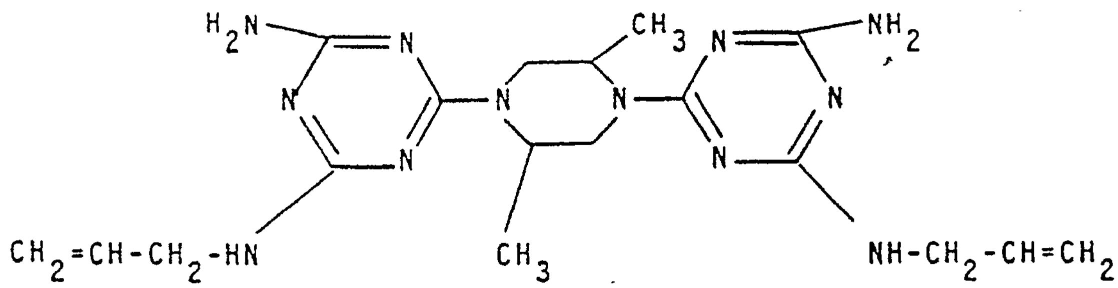

in the form of a white crystalline powder having a m.p. of 192-194 °C.

Example 6

A 2 liter reactor equipped like that of example 3 was charged with 92.2 g of cyanuric acid chloride, 500 ml of acetone and 300 g of ethylenediamine, dissolved in 100 g of water.

Maintaining the temperature at 3-5 °C, 15 g of ethylenediamine, dissolved in 100 g of water, were added over 2 hours. The whole mixture was kept under stirring at 5 °C for 1 hour, then 42 g of solid sodium bicarbonate were added over 1 hour, maintaining the temperature at 5-7 °C. The reaction mass was maintained for 2 hours at 8-10 °C and then was heated to 35 °C.

A solution of 30.6 g of 2-hydroxyethylamine in 100 g of water was added at 8-10 °C over 1 hour.

The temperature was further raised to 40-45 °C, and within 2 hours 20 g of sodium hydroxide in 100 g of water were added. The whole mass was maintained at 50 °C for 1 hour, then acetone distillation was initiated by raising the temperature to 80 °C.

After addition of 81 g of thiomorpholine, the mixture was heated to the reflux temperature. After 2 hours, 16 g of sodium hydroxide in 40 g of water were added and reflux was maintained for a further 6 hours.

After cooling to room temperature, the resulting product was filtered and repeatedly washed with water.

After drying, 96.5 g of product of formula

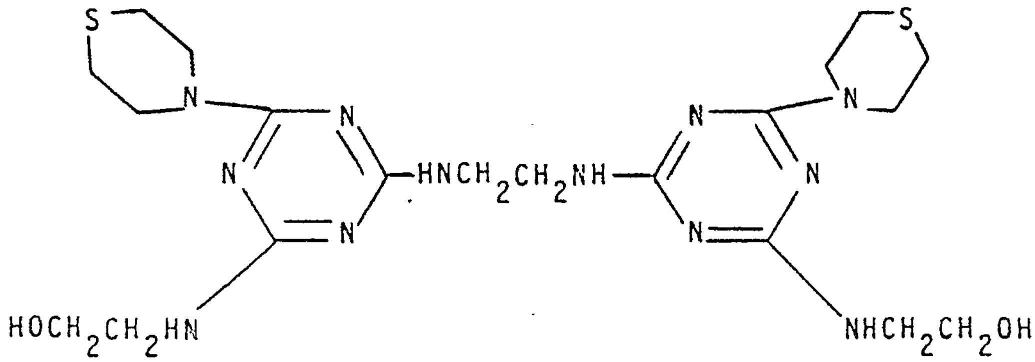

in the form of a white crystalline power having a m. p. higher than 300 °C were obtained.

Example 7

Into a 1 liter reactor equipped as described in example 6, there were placed 92.2 g of cyanuric acid chloride and 300 ml of acetone.

While cooling from the outside at a temperature of 0-5 °C, 21.3 g of piperazine, dissolved in 200 ml of acetone, were added over 1 hour.

Still at a temperature of 0-5 °C, 20 g of sodium hydroxide in 100 g of water were then added.

The whole mixture was stirred at 5 °C for a further 4 hours and then 200 g of cold water were added. The resulting precipitate was filtered and washed with water on the filter.

After drying, 88.7 g of intermediate (XXVIII):

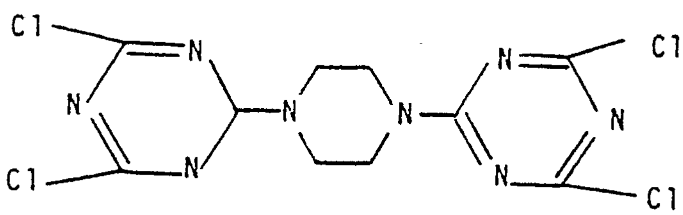

(XXVIII)

in the form of a white crystalline powder having a m.p. higher than 300 °C and a chlorine content equal to 37.4% (theorical: 37.2%) were obtained.

The same 1 liter reactor, but equipped with a heating bath, was charged with 400 ml of xylene and 76.4 g of intermediate (XXVIII).

The mixture was heated to 80 °C and then 69.6 g of morpholine and, subsequently, 32 g of sodium hydroxide in 50 g of water were added within 4 hours.

The temperature was gradually raised by eliminating the water through azeotropic distillation, until reaching the boiling temperature of the solvent.

Reflux was maintained for 8 hours, whereafter the mixture was cooled to room temperature and was filtered ,thoroughly washing the filter cake with water.

After drying, 98.7 g of product of formula

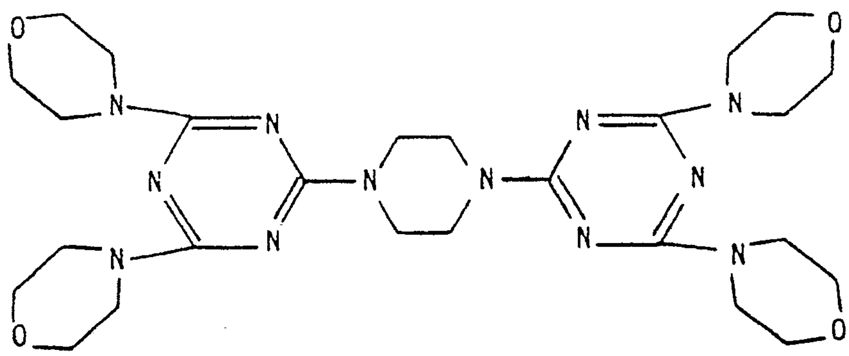

in the form of a white crystalline powder having a m.p. higher than 300 °C were obtained.

Example 8

400 ml of xylene, 64.7 g of intermediate (XXV) and 10.3 g of diethylene triamine were charged to a 1 1-reactor (equipped as disclosed above).

The reaction mixture was heated to 100 °C and kept at this temperature for 2 hours. Then 12 g of sodium hydroxide were added and the reaction mixture was heated to boiling.

The reaction mass was refluxed for 24 hours and then was cooled to room temperature. The precipitated solid product was filtered off and the filter cake was thoroughly washed with water.

By oven-drying at 100 °C, 56.7 g of product of formula:

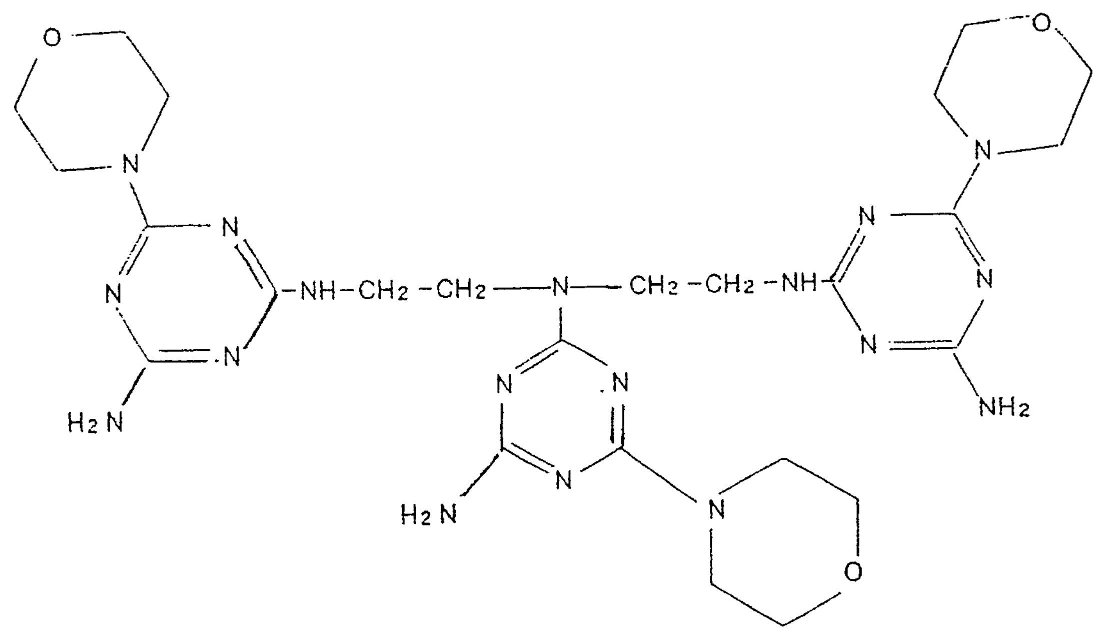

were obtained as crystalline powder having a m.p. of 207-208 °C.

Example 9

500 ml of water, 87.3 g of intermediate (XXIII) and 29.2 g of tris(2-aminoethyl) amine were charged to a 1 1-reactor equipped as disclosed hereinabove.

The reaction mixture was heated to 50 °C and kept at this temperature for 2 hours.

Then 24.0 g of sodium hydroxide, dissolved in 50 ml of water, were added over three hours and the reaction mixture was simultaneously heated to boiling.

The reaction mass was refluxed for about 10 hours and then cooled to room temperature and the

precipitated solid product was filtered off.

The filter cake was washed with water on the same filter and was oven-dried at 100 ° C.

89.4 g of product of formula:

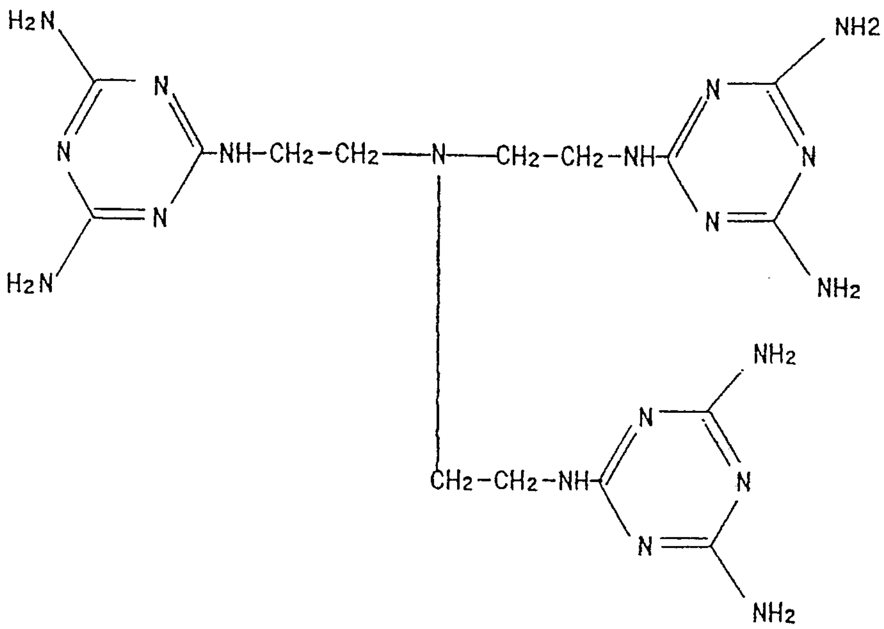

were obtained as white crystalline powder having a m.p. of 125-130.C.

Example 10

184.5 g of cyanuric chloride and 1300 ml of methylene chloride were charged to a 3 1-reactor equipped in the same way as that of example 1.

With the reaction mixture being externally cooled, 75 g of 2-methoxyethylamine and 40 g of sodium hydroxide, dissolved in 150 ml of water, were simultaneously added to the reaction mixture over 3 hours, with the pH value of said reaction mixture being kept within the range of from 5 to 7, and the temperature thereof being kept within the range of from 0 to 3 ° C.

The reaction mixture was kept at a temperature of 0 to 3 ° C for a further 3 hours, and the aqueous phase was then separated.

The organic solution was treated with two 200 ml-portions of water, the aqueous phase being separated each time.

Upon distilling off methylene chloride, 217.5 g of intermediate (XXIX):

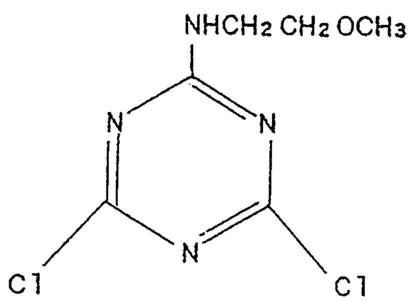

(XXIX)

were obtained as white crystalline powder having a m.p. of 73-75 ° C and a chlorine content of 31.68% (theoretical value: 31.84%).

A 1 1-reactor equipped with stirrer, thermometer, charging funnel, reflux condenser and heating bath,

was charged with 400 ml of acetone and 133.8 g of intermediate (XXIX).

The reaction mixture was heated with stirring to 40 °C until a solution was obtained. Then, with the temperature of the reaction mixture being still kept at 40 °C, 102 g of an aqueous solution of ammonia at 30% by weight were added over 30 minutes.

The resulting reaction mixture was subsequently heated to 45 °C and kept at this temperature for 4 hours.

After cooling to 10 °C, the precipitated solid product was filtered off and the filter cake was washed with cold water on the same filter.

After oven-drying at 100 °C, 114 g of the intermediate product (XXX):

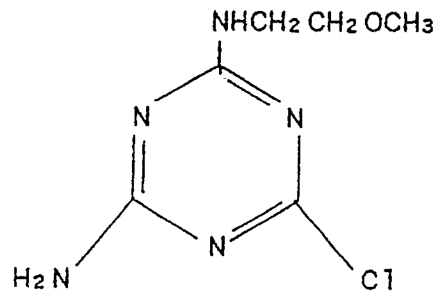

(XXX)

were obtained as crystalline powder having a m.p. of 195-197 °C and a chlorine content of 17.18% (theoretical value: 17.44%).

The structure of the intermediates(XXIX) and (XXX) also was confirmed by I.R.-spectroscopic analyses.

500 ml of ortho-dichlorobenzene, 91.6 g of intermediate (XXX) and 21.9 g of tris(2-aminoethyl) amine were placed in a 1 1-reactor equipped in the same way as that of the preceding example.

The reaction mixture was heated to 100 °C and was kept at that temperature for 2 hours. Then, 18 g of sodium hydroxide were added and the temperature was raised to 140 °C. The reaction mass was kept at 140 °C for 16 hours and then cooled to room temperature, whereafter the precipitated solid product was filtered off. The filter cake was thoroughly washed with water.

After drying the filter cake, 88.2 g of product of formula:

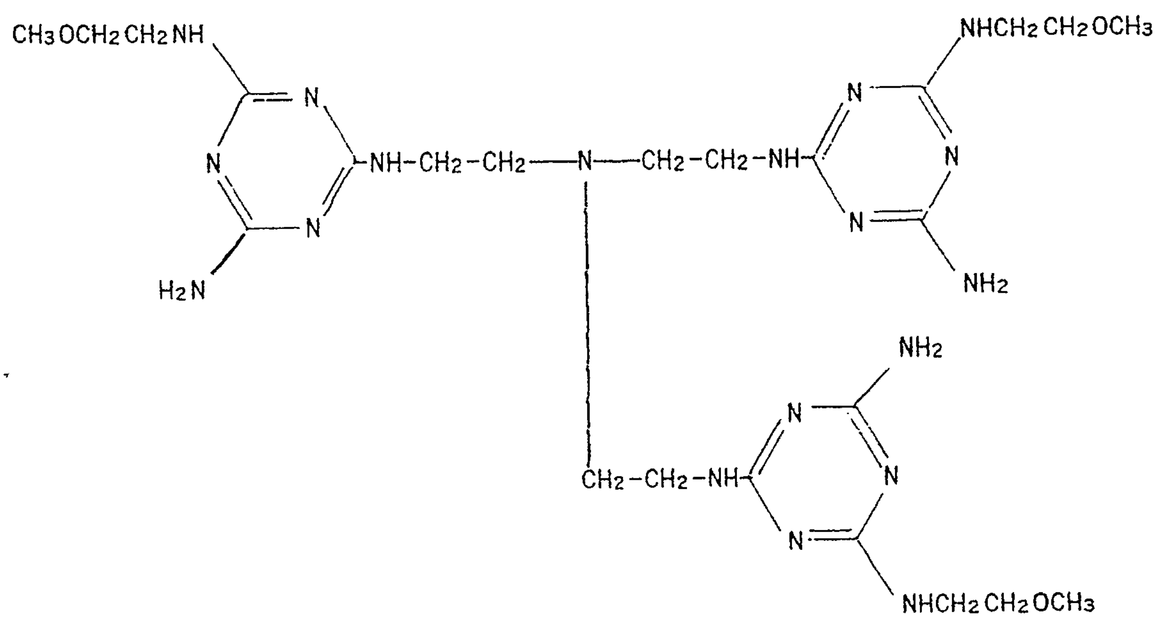

were obtained as crystalline powder having a m.p. of 190-195 °C.

Example 11

500 ml of xylene, 86.2 g of intermediate (XXV) and 15.1 g of tetraethylenepentamine were charged to the 1 1-reactor described in example 10.

The reaction mass was heated to 80 °C and was kept at that temperature for 2 hours. 16 g of sodium hydroxide were then added and the temperature was raised to 110 °C.

The reaction mass was kept at 110 °C for 18 hours and then was cooled to room temperature. The precipitated solid product was filtered off and the filter cake was thoroughly washed with water.

After oven-drying the cake at 100 °C, 82.6 g of product of formula:

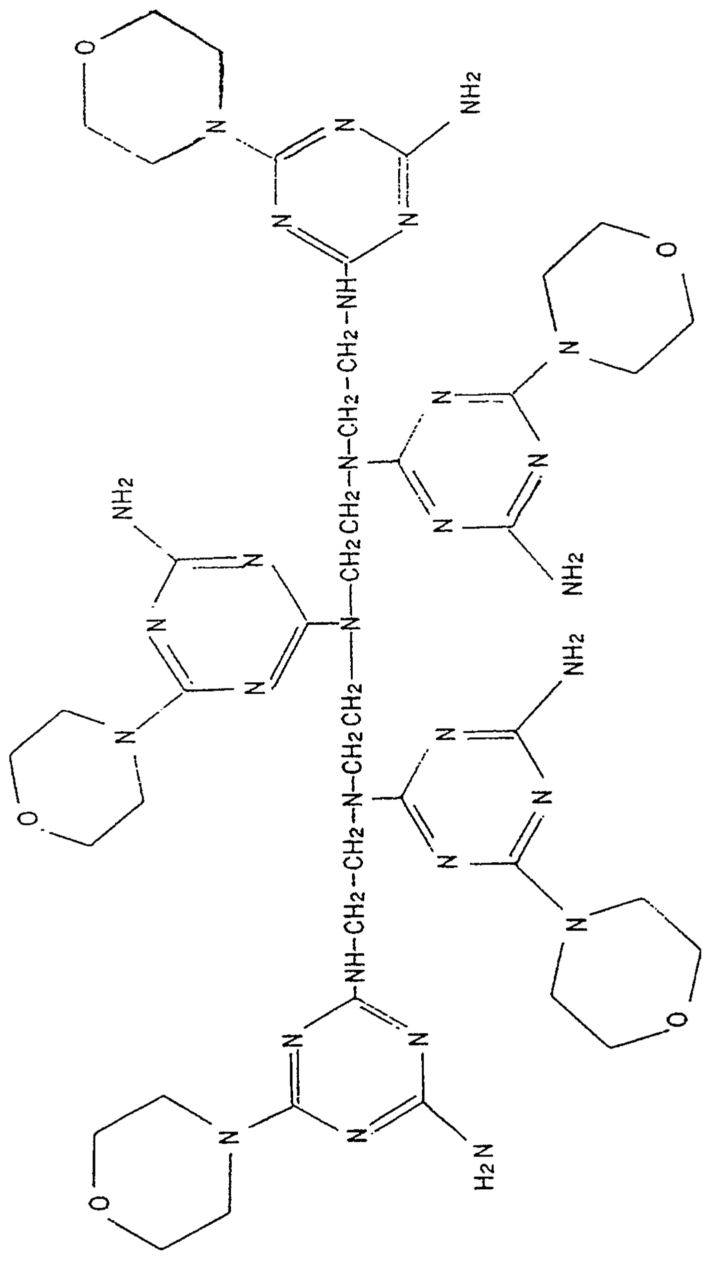

were obtained as crystalline powder having a m.p. of 178-183 °C.

Example 12

55.3 g of cyanuric chloride and 300 ml of acetone were charged to the 1 1-reactor described in example 1.

With the reaction mixture being kept at 0-5° C by means of external cooling, 10.3 g of diethylenetriamine, dissolved in 200 ml of acetone, were added over 1 hour.

With the reaction temperature being still kept at 0 to 5° C, 12 g of sodium hydroxide, dissolved in 100 g of water, were added.

The reaction mixture was stirred, at 5° C, for a further 4 hours and then 200 g of cold water were added. The precipitated solid product was filtered off and the filter cake was washed with water on the same filter.

After drying, 45.6 g of the intermediate (XXXI):

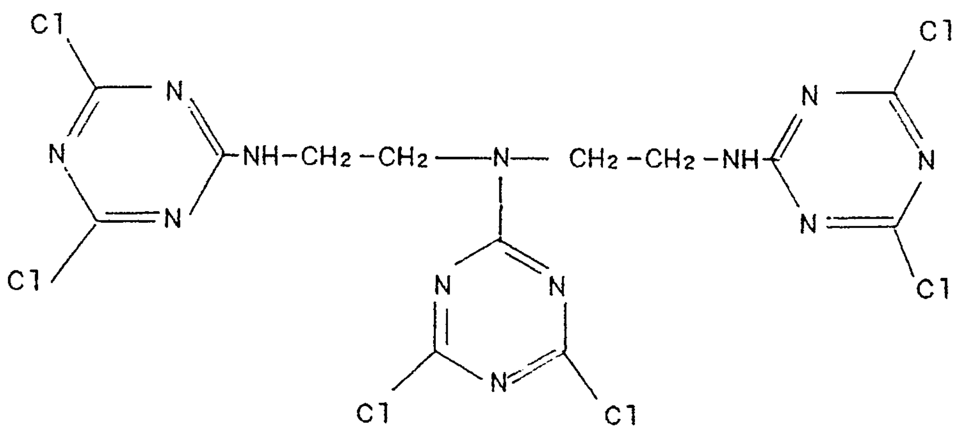

(XXXI)

were obtained as crystalline white powder having a m.p. higher than 300° C and a chlorine content of 38.46% (theoretical value: 38.94%).

To the same 1 1-reactor, this time equipped with a heating bath, 500 ml of xylene and 32.8 g of intermediate (XXXI) were charged.

The reaction mixture was heated to 80° C and subsequently 31.3 g of morpholine and then 14.4 g of sodium hydroxide in 50 g of water were added within 4 hours.

The temperature was gradually increased, with water being removed by azeotropic distillation, until the solvent boiling temperature was reached.

The reaction mixture was kept under reflux conditions for 8 hours and then was cooled to room temperature and filtered. The filter cake was thoroughly washed with water.

After drying, 43.1 g of product of formula:

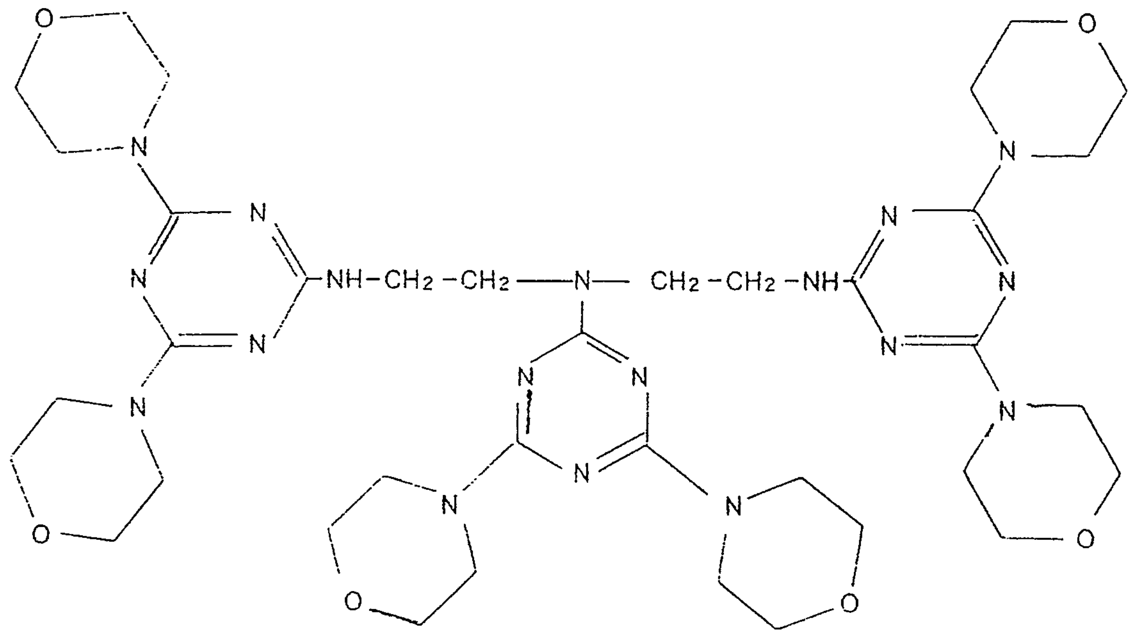

were obtained as white crystalline powder having a m.p. of 277-280 ° C.

Example 13

450 ml of water, 91.6 g of intermediate (XXX) and, with stirring, 15.4 g of diethylenetriamine were charged to the 1 1-reactor of example 10.

The reaction mass was heated to 80 ° C and was kept at that temperature for 3 hours.

18 g of sodium hydroxide, dissolved in 30 ml of water, were then added and the temperature of the reaction mixture was raised to its boiling point. The reaction mass was refluxed for 16 hours and then cooled to about 10 ° C. The precipitated product was filtered off and the filter cake was washed with cold water on the filter.

Upon oven-drying the cake at 100 ° C, 77.9 g of product of formula:

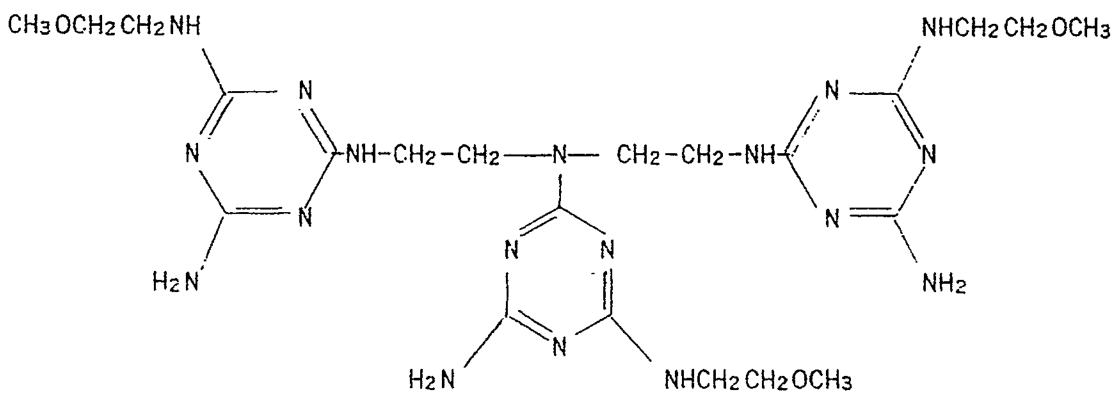

were obtained as crystalline powder having a m.p. of 296-299 ° C.

Example 14

400 ml of water, 86.2 g of intermediate(XXV) and, with stirring, 20.6 g of diethylenetriamine were charged to the 1 1-reactor of example 13.

The reaction mass was heated to 80 ° C and kept at that temperature for 2 hours, whereafter 16 g of

sodium hydroxide, dissolved in 30 ml of water, were added.

The temperature of the reaction mixture was raised to the boiling point and the reaction mass was refluxed for 14 hours.

Then, by operating in the same way as in example 13, 86.2 g of product of formula:

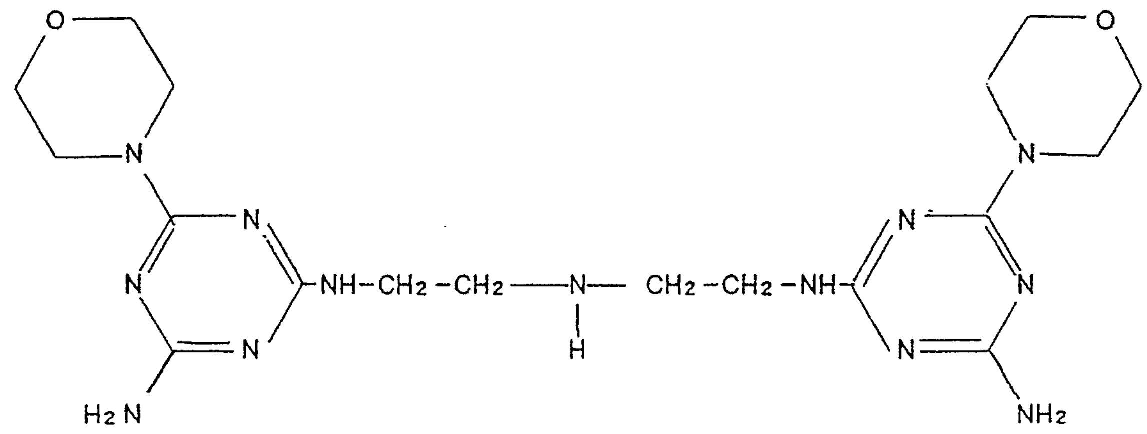

were obtained as crystalline powder having a m.p. of 198-201 °C.

Examples 15-55

By operating under conditions analogous to those disclosed in examples 1 to 14, the compounds of general formula (I) reported in Table 1 were prepared, wherein $R_4$, when present, is a group of formula

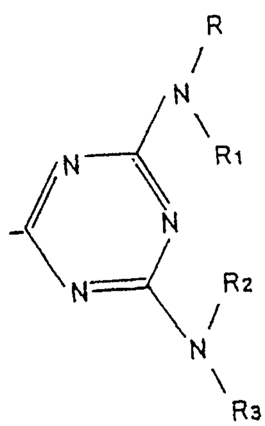

TABLE 1

| EX. No. | $R\text{-}(N)\text{-}R_1$ | | $R_2\text{-}(N)\text{-}R_3$ | | 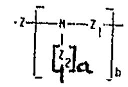 | M.P. (°C) |
|---|---|---|---|---|---|---|
| 15 | 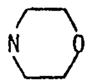 | | H | H | 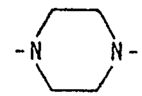 | 276-278 |
| 16 | H | $CH_2CH_2OCH_3$ | H | H | 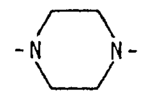 | 212-215 |
| 17 | 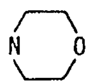 | | H | H | $-NCH_2CH_2NH-$ with $CH_2CH_2OH$ on N | 167-170 |
| 18 | H | H | H | H | $-HNCH_2CH_2NH-$ | > 300 |
| 19 | H | H | H | H | $-HN-(CH_2)_6NH-$ | 273-276 |
| 20 | H | $(CH_2)_3OC_2H_5$ | H | H | 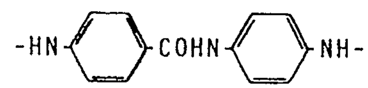 | 226-229 |
| 21 | 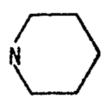 | | H | H | 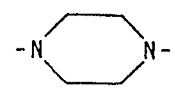 | 272-274 |
| 22 | 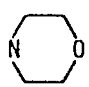 | | H | H | 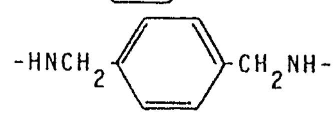 | 240-241 |
| 23 | $CH_2CH_2OH$ | H | H | H | 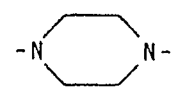 | 252-254 |

TABLE 1 (continued)

| EX. No. | $R-(N)-R_1$ | | $R_2-(N)-R_3$ | | 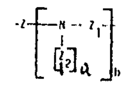 | M.P. (°C) |
|---|---|---|---|---|---|---|
| 24 | $CH_2CH_2OH$ | H | $CH_2CH_2OH$ | H | 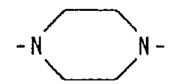 | 236-240 |
| 25 | H | $(CH_2)_5OH$ | H | H | $-HNCH_2CH_2NH-$ | 231-234 |
| 26 | 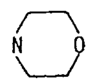 | | H | $CH_2CH_2OH$ | 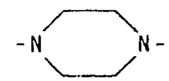 | 260-262 |
| 27 | H | $(CH_2)_3N(C_2H_5)_2$ | H | H | 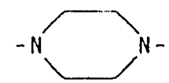 | 281-284 |
| 28 | $(CH_2)_3OH$ | H | H | H | $-NCH_2CH_2N-$ (with $CH_3$ on each N) | 224-227 |
| 29 | 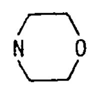 | | H | H | $-NHCH-CH=CH-CHNH-$ (with $CH_3$ on each CH) | 227-229 |
| 30 | $(CH_2)_3OCH_3$ | H | H | H | $-NH-(CH_2)_6N-$ (with $C_6H_{12}$ on N) | 161-163 |
| 31 | $CH_2CH_2OCH_3$ | H | $CH_2CH_2OCH_3$ | H | 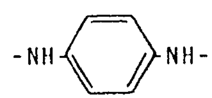 | 170-172 |

T A B L E 1 (continued)

| EX. No. | R-(N)-$R_1$ | $R_2$-(N)-$R_3$ | 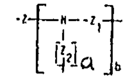 | M.P. (°C) |
|---|---|---|---|---|
| 32 | 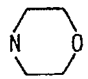 | 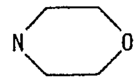 | $-N-CH_2CH_2-N-$ ($CH_2CH_2OH$ on each N: $CH_2CH_2OH$ $CH_2CH_2OH$) | 227-229 |
| 33 | 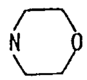 | H H | $-HN(CH_2)_2O-(CH_2)_2NH-$ | 237-240 |
| 34 | 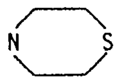 | H H | 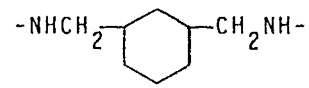 | 142-145 |
| 35 | H $(CH_2)_2O(CH_2)_2OH$ | H H | 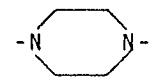 | 202-205 |
| 36 | $CH_3$ $CH_2CH_2OH$ | H H | $-NHCH_2CH_2NH-$ | 230-233 |
| 37 | 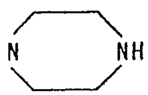 | H H | $-NH-(CH_2)_6NH-$ | 282-284 |
| 38 | 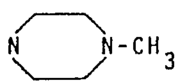 | H H | 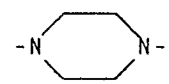 | 278-281 |
| 39 | 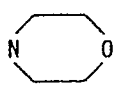 | $t-C_8H_{17}$ H | 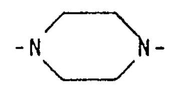 | 187-189 |
| 40 | H $(CH_2)_2OCH=CH_2$ | H H | 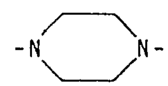 | 216-219 |

TABLE 1 (continued)

| EX. No. | R—(N)—$R_1$ | | $R_2$—(N)—$R_3$ | | 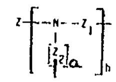 | M.P. (°C) |
|---|---|---|---|---|---|---|
| 41 | n-$C_4H_9$ | H | H | H | 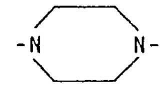 | 174-175 |
| 42 | $CH_2CH_2OCH_3$ | $CH_2CH_2OCH_3$ | H | H | -NH-$(CH_2)_3$NH- | 149-152 |
| 43 | $CH_2CH_2OH$ | H | H | H | 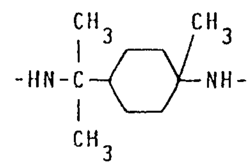 | 215-218 |
| 44 | 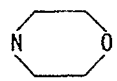 | | H | H | -HN-$(CH_2CH_2O)_2CH_2CH_2$NH- | 172-175 |
| 45 | 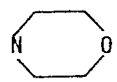 | | H | H | -HN$CH_2CH_2$NH- | 265-268 |
| 46 | $(CH_2)_3OH$ | H | H | H | -N($C_2H_5$)-$CH_2$-CH=CH-$CH_2$-N($C_2H_5$)- | 212-214 |
| 47 | $CH_2CH_2$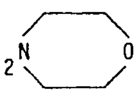 | H | H | H | 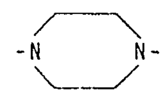 | 274-277 |
| 48 | 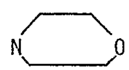 | | H | H | -HN$(CH_2)_3$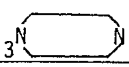$(CH_2)_3$NH- | 220-222 |

TABLE 1 .(continued)

| EX. No. | R-(N)-R$_1$ | | R$_2$-(N)-R$_3$ | | 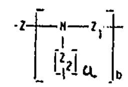 | M.P. (°C) |
|---|---|---|---|---|---|---|
| 49 | $CH_2CH_2OCH_3$ | H | 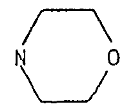 | | $-NH-(CH_2)_3-N-(CH_2)_3-NH-$ | 242-246 |
| 50 | 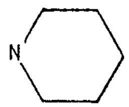 | | H | H | $-NH-(CH_2)_2-N-(CH_2)_2-NH-$ | 259-262 |
| 51 | 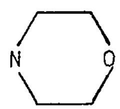 | | $n-C_4H_9$ | H | $-NH-(CH_2)_2-N-(CH_2)_2-NH-$ | 135-142 |
| 52 | $CH_2CH_2CH_2$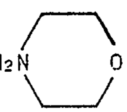 | H | H | H | $N-(CH_2CH_2NH-)_3$ | 172-177 |
| 53 | 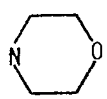 | | $CH_2-CH=CH_2$ | H | $-NH-(CH_2)_4-N-(CH_2)_3-NH-$ | 157-161 |
| 54 | $CH_2CH_2OCH=CH_2$ | H | H | H | $-NH-(CH_2)_3-N-(CH_2)_2-N-(CH_2)_3-NH-$ | 199-204 |
| 55 | H | H | H | H | $-NH-(CH_2)_2-N-(CH_2)_2-NH-$ | 192-196 |

## Examples 56-153

The tests reported in the following Tables 2 and 3 relate to polymeric compositions containing the

product of general formula (I) prepared according to the preceding examples.

Specimen having the shape of small slabs of about 3 mm of thickness were prepared by moulding mixtures of granular polymer and additives on a MOORE platen press, with a moulding cycle of 7 minutes and a moulding pressure of about 40 kg/$cm^2$.

On the slab specimen thus obtained the level of self-extinguishment was determined by measuring the oxygen index (L.O.I. according to ASTM D-2863-77 in a Stanton Redcroft instrument) and by applying the "Vertical Burning Test" which makes it possible to classify the material according to three rating levels (94 V-0, 94 V-1 and 94 V-2) according to the UL 94 standard (issued by Underwriters Laboratories - U.S.A.).

In Table 2 the values which were obtained by using an isotactic polypropylene in flake form and having a melt flow index of 12 and an insoluble fraction in boiling n-heptane of 96% by weight are reported.

In Table 3 there are reported the values which were obtained by using low-density polyethylene in granular form with a melt flow index of 7; polystyrene granules containing 5% by weight of butadiene rubber and having a melt flow index of 9; thermoplastic polyurethane granules, of polyester grade (ESTANE 54600® by Goodrich) or of polyether grade (ESTANE 58300 ® Goodrich), having respective specific gravities of 1.19 and 1.10 g/$cm^3$; an ethylene-propylene elastomeric copolymer containing 45% by weight of propylene; and an acrylonitrile-butadiene-styrene terpolymer having a specific gravity of 1.06 g/$cm^3$ and a melt flow index of 1.6, and containing about 40% of each acrylonitrile and styrene and 20% butadiene.

TABLE 2

| EXAMPLE No. | PRODUCT EXAMPLE No. | PARTS BY WEIGHT PRODUCT | PP (1) | AO (2) | APP (1) | L.O.I. (ASTM D2863) | UL 94 3 mm |
|---|---|---|---|---|---|---|---|
| 56 | 1 | 8.3 | 70 | 1 | 20.7 | 37.6 | V0 |
| 57 | 2 | 6.0 | 75 | 1 | 18.0 | 34.8 | V0 |
| 58 | 3 | 6.0 | 75 | 1 | 18.0 | 34.6 | V0 |
| 59 | 4 | 5.5 | 76 | 1 | 17.5 | 31.2 | V2 |
| 60 | 5 | 9,0 | 64 | 1 | 26.0 | 41.2 | V0 |
| 61 | 6 | 6.0 | 78 | 1 | 15,0 | 31.8 | V0 |
| 62 | 7 | 6.0 | 75 | 1 | 18.0 | 36.3 | V2 |
| 63 | 8 | 6.0 | 81 | 1 | 12.0 | 31.8 | V1 |
| 64 | 8 | 11.3 | 65 | 1 | 22.7 | 45.3 | V0 |
| 65 | 9 | 9.0 | 72 | 1 | 18.0 | 32.2 | V0 |
| 66 | 10 | 8.3 | 70 | 1 | 20.7 | 36.7 | V0 |
| 67 | 11 | 7.0 | 71 | 1 | 21.0 | 37.4 | V0 |
| 68 | 12 | 6.0 | 75 | 1 | 18.0 | 35.4 | V0 |
| 69 | 13 | 8.3 | 74 | 1 | 16.7 | 34.7 | V0 |
| 70 | 14 | 6.8 | 75 | 1 | 17.2 | 32.8 | V0 |
| 71 | 15 | 10.0 | 64 | 1 | 25.0 | 44.5 | V0 |
| 72 | 16 | 5.0 | 75 | 1 | 19.0 | 35.2 | V0 |
| 73 | 17 | 8.0 | 71 | 1 | 20.0 | 36.1 | V0 |
| 74 | 18 | 5.0 | 74 | 1 | 20.0 | 31.7 | V0 |
| 75 | 19 | 5.0 | 77 | 1 | 17.0 | 28.8 | V1 |
| 76 | 20 | 6.0 | 75 | 1 | 18.0 | 30.9 | V0 |
| 77 | 21 | 7.5 | 76 | 1 | 15.5 | 31.7 | V1 |
| 78 | 22 | 8.0 | 71 | 1 | 20.0 | 30.4 | V0 |
| 79 | 23 | 7.0 | 78 | 1 | 14.0 | 33,1 | V0 |
| 80 | 24 | 7.5 | 77 | 1 | 14.5 | 34.5 | V0 |
| 81 | 25 | 6.0 | 75 | 1 | 18.0 | 32.4 | V0 |
| 82 | 26 | 7.0 | 75 | 1 | 17.0 | 36.2 | V0 |
| 83 | 27 | 9.0 | 72 | 1 | 18.0 | 31.2 | V0 |
| 84 | 28 | 6.2 | 75 | 1 | 17.8 | 31.7 | V0 |
| 85 | 29 | 7.0 | 71 | 1 | 21.0 | 32.4 | V0 |
| 86 | 30 | 8,0 | 73 | 1 | 18.0 | 30.2 | V1 |
| 87 | 31 | 6.5 | 73 | 1 | 19.5 | 30.7 | V1 |
| 88 | 32 | 8.5 | 73 | 1 | 17.5 | 32.9 | V1 |
| 89 | 33 | 7.0 | 71 | 1 | 21.0 | 31.6 | V0 |
| 90 | 34 | 7.0 | 71 | 1 | 21.0 | 33.8 | V0 |
| 91 | 35 | 8.0 | 75 | 1 | 16.0 | 32.9 | V0 |
| 92 | 36 | 7.0 | 71 | 1 | 21.0 | 34.9 | V0 |
| 93 | 37 | 8.3 | 74 | 1 | 16.7 | 32.8 | V0 |
| 94 | 38 | 8.3 | 74 | 1 | 16.7 | 32,5 | V0 |
| 95 | 39 | 6.0 | 75 | 1 | 18.0 | 30.8 | V2 |
| 96 | 40 | 8.0 | 75 | 1 | 16.0 | 32.9 | V0 |
| 97 | 41 | 6.0 | 75 | 1 | 18.0 | 30.3 | V1 |
| 98 | 42 | 6.5 | 73 | 1 | 19.5 | 32.0 | V0 |
| 99 | 43 | 9.0 | 72 | 1 | 18.0 | 34.3 | V0 |

TABLE 2 (continued)

| EXAMPLE No. | PRODUCT EXAMPLE No. | PARTS BY WEIGHT PRODUCT | PP (1) | AO (2) | APP (1) | L.O.I. (ASTM D2863) | UL 94 3 mm |
|---|---|---|---|---|---|---|---|
| 100 | 44 | 6.5 | 74 | 1 | 18.5 | 31.2 | V1 |
| 101 | 45 | 6.0 | 75 | 1 | 18.0 | 35.7 | V0 |
| 102 | 46 | 10.0 | 67 | 1 | 22.0 | 37.2 | V0 |
| 103 | 47 | 8.0 | 75 | 1 | 16.0 | 35.1 | V0 |
| 104 | 48 | 6.8 | 75 | 1 | 17.2 | 35.9 | V0 |
| 105 | 49 | 6.8 | 75 | 1 | 17.2 | 35.1 | V0 |
| 106 | 50 | 8.3 | 70 | 1 | 20.7 | 33.2 | V1 |
| 107 | 51 | 7.0 | 71 | 1 | 21.0 | 31.8 | V0 |
| 108 | 52 | 7.4 | 73 | 1 | 18.6 | 32.7 | V0 |
| 109 | 53 | 9.0 | 72 | 1 | 18.0 | 35.6 | V0 |
| 110 | 54 | 8.3 | 74 | 1 | 16.7 | 34.4 | V0 |
| 111 | 55 | 6.5 | 70 | 1 | 22.5 | 34.1 | V0 |
| 112 | 11 | 11.3 | 65 | 1 | * 22.7 | 43.5 | V0 |
| 113 | 13 | 6.8 | 75 | 1 | * 17.2 | 35.3 | V0 |
| 114 | 15 | 7.5 | 74 | 1 | * 17.5 | 36.0 | V0 |
| 115 | 2 | 6.0 | 75 | 1 | 18.0 (3) | 32.9 | V0 |
| 116 | 8 | 7.2 | 74 | 1 | 17.8 (4) | 32.6 | V0 |

(1) PP = polypropylene
APP = ammonium polyphosphate - Exolit 422® (Hoechst)

---------

* APP = ammonium phosphate microencapsulated with melamine formaldehyde resin-Exolit 462® (Hoechst).

---------

(2) AO = antioxidant
a mixture consisting of 2 parts of dilauryl thiopropionate and 1 part of pentaerythritol tetra [3-(3,5-di-tert-butyl-4-hydroxyphenyl)propionate].

---------

(3) = monoammonium salt of 1-aminoethane-1,1-diphosphonic acid.
(4) = monoammonium salt of 1-hydroxyethane-1,1-diphosphonic acid.

TABLE 3

| EX. No. | POLYMERIC SUPPORT (2) | PRODUCT EXAMPLE No. | PARTS BY WEIGHT POLYMER | PRODUCT | AO (3) | APP (1) | L.O.I. (ASTM-D2863) | UL94 3 mm |
|---|---|---|---|---|---|---|---|---|
| 117 | | 2 | 69 | 7.5 | 1 | 22.5 | 27.2 | V1 |
| 118 | | 7 | 64 | 8.0 | 1 | 27.0 | 34.1 | V0 |
| 119 | | 45 | 69 | 7.5 | 1 | 22.5 | 29.2 | V0 |
| 120 | L | 24 | 69 | 8.6 | 1 | 21.4 | 34.8 | V0 |
| 121 | | 15 | 64 | 10.0 | 1 | 25.0 | 32.7 | V0 |
| 122 | D | 16 | 67 | 8.0 | 1 | 24.0 | 34.9 | V0 |
| 123 | | 3 | 64 | 10.0 | 1 | 25.0 | 33.8 | V1 |
| 124 | P | 26 | 60 | 11.0 | 1 | 28.0 | 43.6 | V0 |
| 125 | | 23 | 71 | 8.0 | 1 | 20.0 | 30.6 | V1 |
| 126 | E | 8 | 60 | 10.0 | 1 | 29.0 | 39.0 | V0 |
| 127 | | 10 | 67 | 8.0 | 1 | 24.0 | 34.9 | V0 |
| 128 | | 11 | 70 | 7.4 | 1 | 21.6 | 33.6 | V0 |
| 129 | | 48 | 65 | 9.7 | 1 | 24.3 | 34.7 | V0 |
| 130 | | 53 | 69 | 7.5 | 1 | 22.5 | 32.8 | V0 |
| 131 | | 24 | 64 | 11.7 | 1 | 23.3 | 31.8 | V0 |
| 132 | | 15 | 65 | 11.7 | 1 | 23.3 | 32.6 | V1 |
| 133 | H | 16 | 64 | 10.0 | 1 | 25.0 | 31.0 | V0 |
| 134 | I | 26 | 59 | 10.0 | 1 | 30.0 | 38.0 | V0 |
| 135 | P | 23 | 60 | 13.0 | 1 | 26.0 | 31.2 | V0 |
| 136 | S | 8 | 63 | 9.0 | 1 | 27.0 | 31.7 | V0 |
| 137 | | 10 | 64 | 10.0 | 1 | 25.0 | 32.0 | V0 |
| 138 | | 48 | 67 | 9.0 | 1 | 23.0 | 30.8 | V1 |
| 139 | | 15 | 70 | 8.2 | 1 | 20.8 | 32.4 | V0 |
| 140 | PU | 17 | 70 | 8.0 | 1 | 21.0 | 34.1 | V0 |
| 141 | | 8 | 70 | 8.0 | 1 | 21.0 | 34.1 | V0 |
| 142 | ester | 11 | 70 | 8.2 | 1 | 20.8 | 32.4 | V0 |
| 143 | | 48 | 67 | 9.0 | 1 | 23.0 | 33.2 | V0 |
| 144 | PU (e | 3 | 66 | 10.0 | 1 | 23.0 | 30.6 | V0 |
| 145 | ther) | 8 | 67 | 9.0 | 1 | 23.0 | 30.4 | V0 |
| 146 | | 15 | 60 | 11.0 | 1 | 28.0 | 36.8 | V0 |
| 147 | | 26 | 60 | 10.0 | 1 | 29.0 | 37.2 | V0 |
| 148 | PP/PE | 8 | 63 | 9.0 | 1 | 27.0 | 40.1 | V0 |
| 149 | | 13 | 60 | 11.0 | 1 | 28.0 | 38.7 | V0 |
| 150 | | 48 | 59 | 20.0 | 1 | 20.0 | 37.0 | V0 |
| 151 | | 15 | 65 | 11.0 | 1 | 23.0 | 30.4 | V0 |
| 152 | ABS | 24 | 64 | 10.0 | 1 | 25.0 | 32.2 | V0 |
| 153 | | 48 | 64 | 10.0 | 1 | 25.0 | 31.8 | V0 |

Footnotes to Table 3

(1) APP = ammonium polyphosphate
(2) LDPE = low-density polyethylene
HIPS = polystyrene containing 5% of butadiene rubber
(Ester) PU = polyester polyurethane
(Ether) PU = polyether polyurethane
PP/PE = propylene-ethylene copolymer
ABS = acrylonitrile-butadiene-styrene terpolymer
(3) AO = antioxidant: a mixture consisting of 2 parts of dilauryl thiopropionate and 1 part of pentaerythritol tetra [3-(3,5-di-tert butyl-4-hydroxyphenyl) propionate]

Example No. 154 (Comparative Example)

By following the same procedure as in examples 56 to 116, but with 2,4,6-triamino-1,3,5-triazine being used as the nitrogen-containing compound, the following composition was prepared:

| | |
|---|---|
| - Polypropylene : | 72 parts by weight |
| - Antioxidant : | 1 part by weight |
| - Ammonium polyphosphate : | 18 parts by weight |
| - 2,4,6-triamino-1,3,5-triazine : | 9 parts by weight |

By using the above composition, specimen were prepared and tested for their self- extinguishing properties, according to the method disclosed hereinabove.

The following results were obtained:

L.O.I. : 25.2

UL 94 (3 mm) : Class B (the specimen burns).

**Claims**

1. Polymeric self-extinguishing compositions comprising:

(a) from 89 to 45 parts by weight of thermoplastic polymer and/or of polymer endowed with elastomeric properties;

(b) from 8 to 30 parts by weight of one or more compounds selected from ammonium and amine phosphates and ammonium and amine phosphonates;

(c) from 3 to 25 parts by weight of one or more 2,4,6-triamino-1,3,5-triazine derivatives of general formula (I):

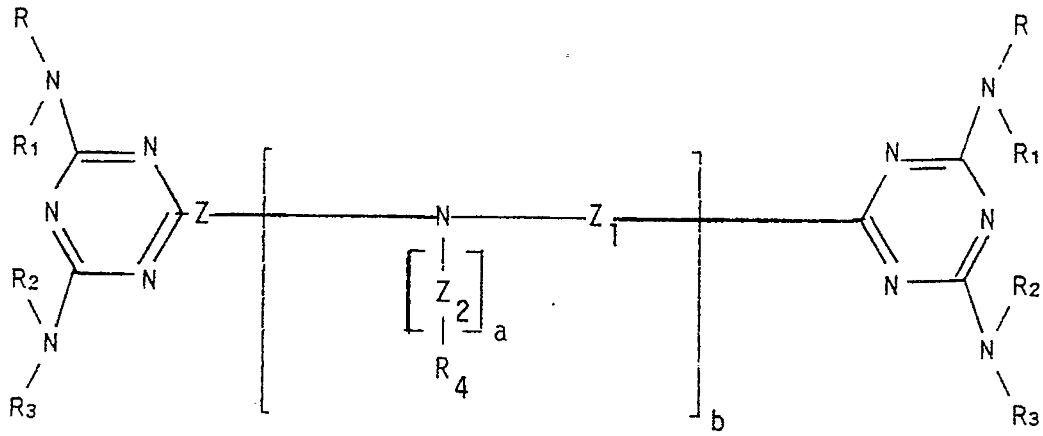

(I)

wherein the radicals R, $R_1$, $R_2$ and $R_3$, equal to or different from one another and having the same or different meanings in each triazine ring, are selected from H; ($C_1$-$C_{18}$)-alkyl; ($C_2$-$C_8$)-alkenyl; ($C_6$-$C_{16}$)-cycloalkyl and ($C_6$-$C_{16}$)-alkylcycloalkyl, optionally substituted with one or more hydroxy and/or ($C_1$-$C_4$)-hydroxyalkyl groups;

$\{C_nH_{2n}\}O$-$R_5$; and

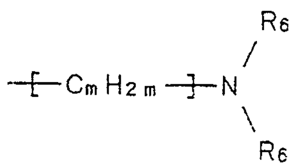

;

wherein:

n = an integer of from 2 to 8;

m = an integer of from 2 to 6;

$R_5$ = H; ($C_1$-$C_8$)-alkyl; ($C_2$-$C_6$)-alkenyl; $\{C_pH_{2p}\}O$-$R_7$, wherein p is an integer of from 1 to 4 and $R_7$ is H or a ($C_1$-$C_4$)-alkyl group; ($C_6$-$C_{12}$)-cycloalkyl; or ($C_6$-$C_{12}$)-alkylcycloalkyl;

the radicals $R_6$, equal to or different from one another, are selected from H; ($C_1$-$C_8$)-alkyl; ($C_2$-$C_6$)-alkenyli ($C_6$-$C_{12}$)-cycloalkyl; or ($C_6$-$C_{12}$)-alkylcycloalkyl; and (Cl-$C_4$)-hydroxyalkyl;

or the moiety $N(R_6)_2$ is replaced by an N-heterocyclic radical which optionally contains another heteroatom and is linked to the alkyl chain through the nitrogen atom;

or in general formula (I) at least one of the moieties

$NRR_1$ and $NR_2R_3$

is replaced by an N-heterocyclic radical which optionally contains another heteroatom and is linked to the triazine ring through the nitrogen atom;

a is 0 or 1;

b is 0 or an integer of from 1 to 5;

$R_4$ is hydrogen or a group of general formula

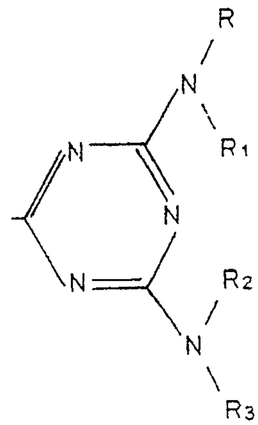

and its meaning can be different in each repeating unit;

when b is zero:

Z is selected from radicals of the following formulae:

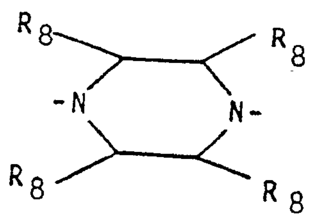

(II)

where the groups $R_8$, the same or different from one another, represent hydrogen or $(C_1-C_4)$-alkyl;

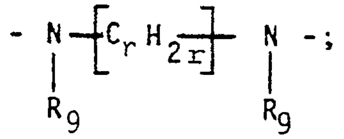

(III)

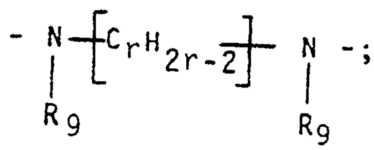

(IV)

where r is an integer of from 2 to 14 and $R_9$ is hydrogen; $(C_1-C_4)$-alkyl; $(C_2-C_6)$-alkenyl; or $(C_1-C_4)$-hydroxyalkyl;

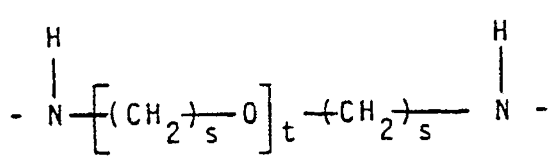

(VI)

where s is an integer of from 2 to 5 and t is an integer of from 1 to 3;

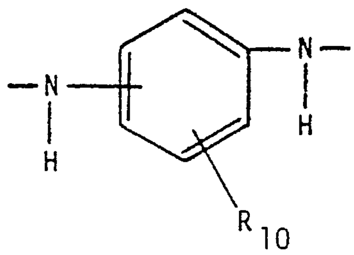

(VII)

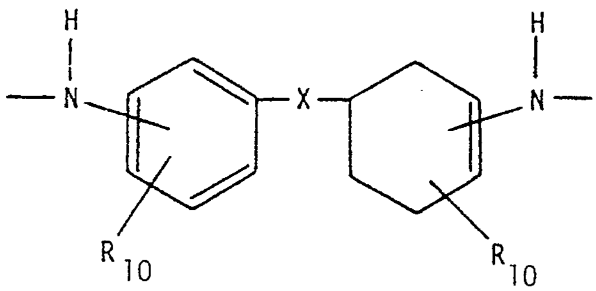

(VIII)

where:

X represents a direct bond; O; S; S-S; SO; $SO_2$; NH; $NHSO_2$; NHCO; N=N; and $CH_2$;

$R_{10}$ is hydrogen; hydroxy; $(C_1-C_4)$-alkyl; or $(C_1-C_4)$-alkoxy;

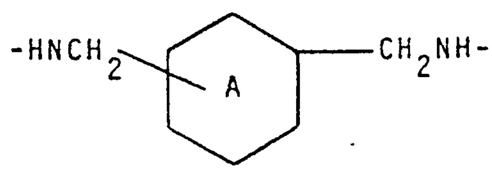

(IX)

where A is a saturated or unsaturated ring;

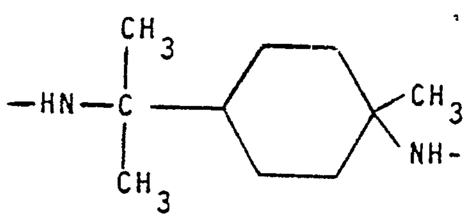

(X)

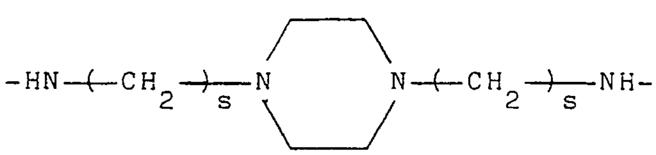

(XI)

where s has the meaning defined hereinabove;

when b is an integer of from 1 to 5:

the group:

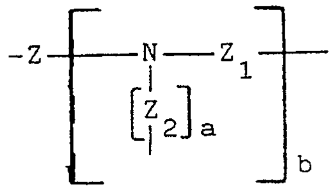

is a polyvalent radical represented by one of the following formulae:

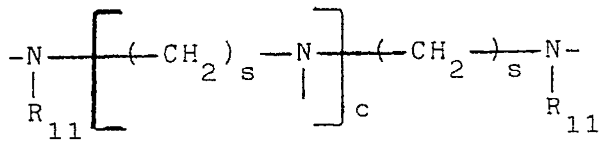

(XII)

where:
$R_{11}$ is hydrogen or ($C_1$-$C_4$)-alkyl;
c is an integer of from 1 to 5;
the subscripts s, equal to or different from each other, have the meaning defined hereinabove; and

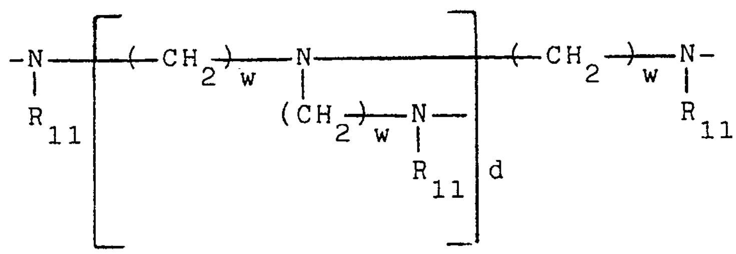

(XIII)

where:
$R_{11}$ has the meaning defined hereinabove;
w is an integer of from 2 to 4; and
d is either 1 or 2.

2. Compositions according to claim 1, wherein at least one of the moieties
$-NRR_1$ and $-NR_2R_3$
in general formula (I) is replaced by a heterocyclic radical selected from aziridinyl, pyrrolidinyl, piperidinyl, morpholinyl, thiomorpholinyl, piperazinyl, 4-methylpiperazinyl, 4-ethylpiperazinyl, 2-methylpiperaziny, 2,5-dimethylpiperazinyl, 2,3,5,6-tetramethylpiperazinyl, 2,2,5,5-tetramethylpiperazinyl, 2-ethylpiperazinyl and 2,5-diethylpiperazinyl.

3. Compositions according to any one of claims 1 and 2, wherein at least one of the radicals R, $R_1$, $R_2$ and $R_3$ in general formula (I) is a radical of formula

$$-[C_nH_{2n}]-O-R_5$$

wherein:
n is an integer of from 2 to 4; and
$R_5$ is H or ($C_1$-$C_4$)-alkyl.

4. Compositions according to any one of claims 1 to 3, wherein at least one of the moieties
$-NRR_1$ and $-NR_2R_3$
in general formula (I) is an $-NH_2$ radical.

5. Compositions according to any one of the preceding claims, wherein the moiety
$-N(R_6)_2$
in general formula (I) is replaced by a heterocyclic radical selected from aziridinyl, pyrrolidinyl, piperidinyl, morpholinyl, thiomorpholinyl, piperazinyl, 4-methylpiperazinyl, and 4-ethylpiperazinyl.

6. Compositions according to any one of the preceding claims, wherein the ammonium phosphates (b) have

the general formula

$(NH_4)_{n+2}P_nO_{3n+1}$

wherein n is an integer of at least 2; or have the general formula

$(NH_4PO_3)_n$

wherein n ranges from 50 to 500.

7. Compositions according to any of claims 1 to 5, wherein the amine phosphates (b) are selected from diethylammonium phosphate, diethylamine phosphate, ethylene diamine phosphate, melamine orthophosphate and melamine pyrophosphate.

8. Compositions according to any of claims 1 to 5, wherein the mono- or polyammonium phosphonates (b) are selected from the salts derived from mono- or polyphosphonic acids.

9. Compositions according to any of claims 1 to 8, wherein the polymer (a) is selected from acrylonitrile/butadiene/styrene terpolymers; styrene/acrylonitrile copolymers; polyurethanes; poly-(ethyleneterephthalate); poly(butylene terephthalate), polyamides; and polymers and copolymers of olefins of general formula

$R' - CH = CH_2$

wherein $R'$ is a hydrogen atom or a $(C_{1-8})$-alkyl or $(C_{6-10})$-aryl radical,

particularly:

(1) isotactic or prevailingly isotactic polypropylene;

(2) HDPE, LLDPE and LDPE polyethylene;

(3) crystalline copolymers of propylene and minor amounts of ethylene and/or other alpha-olefins, such as, e.g., 1-butene, 1-hexene, 1-octene and 4-methyl-1-pentene;

(4) heterophasic compositions comprising (A) a fraction constituted by a propylene homopolymer or one of the copolymers specified under item (3) above; and (B) a copolymeric fraction constituted by elastomeric copolymers of ethylene and an alpha-olefin, optionally containing minor amounts of diene, wherein the alpha-olefin is preferably selected from propylene and 1-butene; and

(5) elastomeric copolymers of ethylene and alpha-olefin(s), optionally containing minor amounts of diene.

10) Moulded articles obtained from the compositions according to any one of the preceding claims.